# EUROPEAN PATENT APPLICATION

(11) **EP 1 935 886 A1**
(43) Date of publication of application: **25.06.2008**
(21) Application number: 06384019.3
(22) Date of filing: 22.12.2006
(51) Int. Cl.: C07D 231/12, C07D 261/08, C07C 211/27, A61K 31/415, A61K 31/42, A61K 31/137, A61P 25/00

(54) **Heterocyclyl-substituted-ethylamino-phenyl derivatives, their preparation and use as medicaments**

(71) Applicant: Laboratorios del Dr. Esteve S.A., 08041 Barcelona (ES)
(72) Inventor: Garcia-Lopez, Monica., 08018 Barcelona (ES); Torrens- Jover, Antonio., 08221 Terrassa (Barcelona) (ES); Romero-Alonso, Luz., 08904 L`Hospilatet de Llobregat, (Barcelona) (ES); Buschmann, Helmut. H., 08960 Sant Just Desvern, ( Barcelona ) (ES)
(74) Representative: Peters, Hajo

(57) **Abstract**

The present invention relates to heterocyclyl-substituted-ethylamino-phenyl compounds of general formula (I) wherein K-L-M-N, Z and R¹, R², R³, R⁴ are described in the claims, methods for their preparation, medicaments comprising these compounds as well as their use for the preparation of a medicament for the treatments of humans or animals.

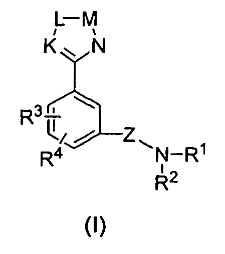

## Description

The present invention relates to heterocyclyl-substituted-ethylamino-phenyl compounds of general formula (I), methods for their preparation, medicaments comprising these compounds as well as their use for the preparation of a medicament for the treatment of humans or animals

The search for new therapeutic agents has been greatly aided in recent years by better understanding of the structure of proteins and other biomolecules associated with target diseases. One important class of proteins that has been the subject of extensive study is the family of 5-hydroxytryptamine (serotonin, 5-HT) receptors. The 5-HT₇ receptor discovered in 1993 belongs to this family and has attracted great interest as a valuable new drug target (Terrón, J.A. Idrugs, 1998, vol. 1, no. 3, pages 302-310: "The 5HT7 receptor: A target for novel therapeutic avenues?*"* ).

5-HT₇ receptors have been cloned from rat, mouse, guinea pig and human cDNA and exhibit a high degree of interspecies homology (approx. 95%), but it is unique in that it has a low sequence homology with other 5-HT receptors (less than 40%). Its expression pattern, in particular structures of the central nervous system (CNS) (highest in hypothalamus (in particular suprachiasmatic nuclei) and thalamus) and other peripheral tissues (spleen, kidney, intestinal, heart and coronary arthery), implicates the 5-HT₇ receptor in a variety of functions and pathologies. This idea is reinforced by the fact that several therapeutic agents, such as tricyclic antidepressants, typical and atypical antipsychotics and some 5-HT₂ receptor antagonists, display moderate to high affinity for both recombinant and functional 5-HT₇ receptors.

Functionally, the 5-HT₇ receptor has been implicated in regulation of circadian rhythms in mammals (Lovenberg, T.W. et al. Neuron, 1993, 11:449-458 "A novel adenylyl cyclase-activating serotonin receptor (5-HT7) implicated in the regulation of circadian rhythms*").* It is known that disruption of circadian rhythms is related to a number of CNS disorders including depression, seasonal affective disorder, sleep disorders, shift worker syndrome and jet lag among others.

Distribution and early pharmacological data also suggest that the 5-HT₇ receptor is involved in the vasodilatation of blood vessels. This has been demonstrated *in vivo* (Terrón, J.A., Br J Pharmacol, 1997, 121:563-571 "Role of 5-HT7 receptors in the long lasting hypotensive response induced by 5-hydroxytryptamine in the rat*"*)*.* Thus selective 5-HT₇ receptor agonists have a potential as novel hypertensive agents.

The 5-HT₇ receptor has also been related with the pathophysiology of migraine through smooth muscle relaxation of cerebral vessels (Schoeffter, P. et al., 1996, Br J Pharmacol, 117:993-994; Terrón, J.A., 2002, Eur. J. Pharmacol., 439:1-11 "Is the 5-HT7 receptor involved in the pathogenesis and prophylactic treatment of migraine?*"*). In a similar manner, involvement of 5-HT₇ in intestinal and colon tissue smooth muscle relaxation makes this receptor a target for the treatment of irritable bowel syndrome (De Ponti, F. et al., 2001, Drugs, 61:317-332 "Irritable bowel syndrome. New agents targeting serotonin receptor subtypes*"*). Recently, it has also been related to urinary incontinence *(*British J. of Pharmacology, Sept. 2003, 140(1) 53-60: "Evidence for the involvement of central 5HT-7 receptors in the micurition reflex in anaeshetized female rats*"*).

In view of the potential therapeutic applications of agonists or antagonists of the 5HT₇ receptor, a great effort has been directed to find selective ligands. Despite intense research efforts in this area, very few compounds with selective 5-HT₇ antagonist activity have been reported (Wesolowska, A., Polish J. Pharmacol., 2002, 54: 327-341, "In the search for selective ligands of 5-HT5, 5-HT6 and 5-HT7 serotonin receptors'*),* yet even fewer 5-HT7-Agonists.

There is still a need to find compounds that have pharmacological activity towards the receptor 5-HT₇, being both effective and selective, and having good "drugability" properties, i.e. good pharmaceutical properties related to administration, distribution, metabolism and excretion.

Thus, it was an object of the present invention to provide novel compounds that are suitable in particular as active substances in medicaments.

Said object was achieved by providing a heterocyclyl-substituted-ethylamino-phenyl derivative of general formula (I) 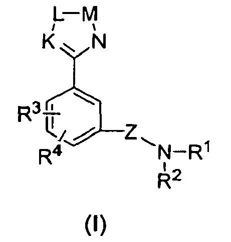 wherein
K-L-M-N together form
- =CH-X-Y=CH-; in which any suitable H may be substituted by R⁶ and/or R⁷, and in which X is selected from NR⁸, O or S, while Y is selected from N or CH;
- =CH-X-Y-C(O)-; in which any suitable H may be substituted by R⁶ and in which one of X and Y is NR⁸, while the other is selected from NR^{8a}, S or O;
- =CH-X-Y-C(O)-; in which one of X and Y is CH₂, while the other is selected from NR⁸, S or O, in which any suitable H may be substituted by R⁶ and/or R⁷;
- =CR⁶-N=N-C(O)-;
- =CR⁹-CH=CH-CH=CH-; in which any suitable H may be substituted by R⁶;
- =CR⁹-CH=CH-CH=CR^{9a}-; in which any suitable H may be substituted by R⁶;
- =CH-X=Y-CH=CH-; in which any suitable H may be substituted by R⁶ and/or R⁷, and in which one of X or Y is selected from N, while the other is selected from N or CH;
- =CH-X=Y-CH₂-CH₂-; in which any suitable H may be substituted by R⁶ and/or R⁷, and in which one of X or Y is selected from N, while the other is selected from N or CH;
- =CH-X-Y-CH=CH-; in which any suitable H may be substituted by R⁶ and/or R⁷, and in which one of X or Y is selected from NR⁸, O or S while the other is selected from NR^{8a} or CH₂;
- =CH-X-Y-CH₂-CH₂-; in which any suitable H may be substituted by R⁶ and/or R⁷, and in which one of X or Y is selected from NR⁸, O or S while the other is selected from NR^{8a} or CH₂;
- =CH-X-CH₂-Y=CH-; in which any suitable H may be substituted by R⁶ and/or R⁷, and in which X is selected from NR⁸, O or S while Y is selected from N or CH;
- =CH-X-CH=Y-CH₂-; in which any suitable H may be substituted by R⁶ and/or R⁷, and in which X is selected from NR⁸, O or S while Y is selected from N or CH;
- =CH-N=CH-Y=CH-; in which any suitable H may be substituted by R⁶ and/or R⁷;
- =CH-X-CH₂-Y-CH₂-; in which any suitable H may be substituted by R⁶ and/or R⁷, and in which one of X or Y is selected from NR⁸, O or S while the other is selected from NR^{8a}, O, S or CH₂;

R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem;
Z is selected from
   - -(CH₂)ₙ-, with n being 1, 2, 3 or 4;
   - -O-(CH₂)ₙ-, with n being 1, 2, 3 or 4;
   - -S-(CH₂)ₙ-, with n being 1, 2, 3 or 4;
   - (CH₂)ₙ-(CHR⁵)-(CH₂)ₘ, with n and m being selected from 0, 1, 2 or 3 and m+n being 1, 2 or 3, with R⁵ being selected from F, Cl, Br, I, OH, SH, or unsubstituted C₁₋₄-Alkyl;
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively.

In a preferred embodiment the following proviso(s)/disclaimer(s) applies:
- with the proviso that
   if R¹and R² are both CH₃, R³ and R⁴ are both H, K-L-M-N together form =CR⁹-CH=CH-CH=CR^{9a}- and one of R⁹ or R^{9a} is -CH=CH₂, the other may not be OCH₃;
   and/or
- with the proviso that
   if R¹ and R² are both H, one of R³ and R⁴ is H, while the other is -O(C₂H₅), K-L-M-N together form =CR⁹-CR⁶=CH-CH=CH-, and R⁹ is -OCH₃, R⁶ may not be OCH₃.

These compounds show a high affinity to the 5HT₇ Receptor as well as a high selectivity for this receptor in comparison to e.g. the 5HT₆, the Sigma 1, the α2, and the 5HT₁ Receptor, thus having a higher affinity to the 5HT₇ receptor. In addition some of these compounds show an agonistic activity on this receptor.

A "mono- or polycyclic ring-system" according to the present invention means a mono- or polycyclic hydrocarbon ring-system that may be saturated, unsaturated or aromatic. If the ring system is polycyclic, each of its different rings may show a different degree of saturation, i.e. it may be saturated, unsaturated or aromatic. Optionally each of the rings of the mono- or polycyclic ring system may contain one or more heteroatoms as ring members, which may be identical or different and which can preferably be selected from the group consisting of N, O, S and P, more preferably be selected from the group consisting of N, O and S. Preferably the polycyclic ring-system may comprise two rings that are condensed. The rings of the mono- or polycyclic ring-sytem are preferably 5- or 6-membered.

An "aryl", "aryl radical" or group is understood as meaning ring systems with at least one aromatic ring but without heteroatoms even in only one of the rings. Examples are phenyl, naphthyl, fluoranthenyl, fluorenyl, tetralinyl or indanyl, in particular 9H-fluorenyl or anthracenyl radicals, which can be unsubstituted or monosubstituted or polysubstituted.

In the context of this invention "cycloalkyl radical" or group is understood as meaning saturated and unsaturated (but not aromatic) cyclic hydrocarbons (without a heteroatom in the ring), which can be unsubstituted or mono- or polysubstituted. Furthermore, C₃₋₄-cycloalkyl represents C₃- or C₄-cycloalkyl, C₃₋₅-cycloalkyl represents C₃-, C₄- or C₅-cycloalkyl, C₃₋₆-cycloalkyl represents C₃-, C₄-, C₅- or C₆-cycloalkyl, C₃₋₇-cycloalkyl represents C₃-, C₄-, C₅-, C₆- or C₇-cycloalkyl, C₃₋₈-cycloalkyl represents C₃-, C₄-, C₅-, C₆-, C₇- or C₈-cycloalkyl, C₄-₅-cycloalkyl represents C₄- or C₅-cycloalkyl, C₄₋₆-cycloalkyl represents C₄-, C₅- or C₆-cycloalkyl, C₄₋₇-cycloalkyl represents C₄-, C₅-, C₆- or C₇-cycloalkyl, C₄₋₈-cycloalkyl represents C₄-, C₅-, C₆- C₇- or C₈-cycloalkyl C₅₋₆-cycloalkyl represents C₅- or C₆-cycloalkyl and C₅₋₇-cycloalkyl represents C₅-, C₆- or C₇-cycloalkyl. However, mono- or polyunsaturated, preferably monounsaturated, cycloalkyls also in particular fall under the term cycloalkyl as long as the cycloalkyl is not an aromatic system. The cycloalkyl radicals are preferably cyclopropyl, 2-methylcyclopropyl, cyclopropylmethyl, cyclobutyl, cyclopentyl, cyclopentylmethyl, cyclohexyl, cycloheptyl, cyclooctyl, and also adamantly.

A "heterocyclyl", a "heterocyclyl radical" or group or "heterocyclic ring system" is understood as meaning heterocyclic ring systems which contain one or more heteroatoms from the group consisting of nitrogen, oxygen and/or sulfur in the ring or ringsystem, and can also be mono- or polysubstituted. The ringsystem may consist either of only one saturated or unsaturated or even aromatic ring or may consist of 2, 3 or 4 saturated or unsaturated or even aromatic rings, which are condensed in that between two or more of the rings ring members are shared. Examples which may be mentioned from the group of heterocyclyls are furan, benzofuran, thiophene, benzothiophene, pyrrole, pyridine, pyrimidine, pyrazine, quinoline, isoquinoline, phthalazine, benzo-1,2,5-thiadiazole, imidazo-thiazole, benzothiazole, indole, benzotriazole, benzodioxolane, benzodioxane, carbazole and quinazoline.

In connection with mono- or polycyclic ring-system, aryl radical, cycloalkyl radical, or heterocyclyl radical, "substituted" is understood - unless defined otherwise - as meaning replacement of at least one hydrogen radical on the ring-system of the mono- or polycyclic ring-system, the aryl radical, the cycloalkyl radical, or the heterocyclyl radical by OH, SH, =O, halogen (F, Cl, Br, I), CN, NO₂. COOH; NRₓR_{y}, with Rₓ and R_{y} independently being either H or a saturated or unsaturated, linear or branched, substituted or unsubstituted C₁₋₆-alkyl; by a saturated or unsaturated, linear or branched, substituted or unsubstituted C₁₋₆-alkyl; a saturated or unsaturated, linear or branched, substituted or unsubstituted -O-C₁₋₆-alkyl (alkoxy); a saturated or unsaturated, linear or branched, substituted or unsubstituted -S-C₁₋₆-alkyl; a saturated or unsaturated, linear or branched, substituted or unsubstituted -C(O)-C₁₋₆-alkyl; a saturated or unsaturated, linear or branched, substituted or unsubstituted -C(O)-O-C₁₋₆-alkyl; a substituted or unsubstituted phenyl. Within that "monosubstituted" means the substitution of exactly one hydrogen radical, whereas "polysubstituted" means the substitution of more than one hydrogen radical with "polysubstituted"radicals being understood as meaning that the replacement takes effect both on different and on the same atoms several times with the same or different substituents. Therefore, "optionally at least monsubstituted" means either "not substituted" if the option is not fulfilled, "monosubstituted" or "polysubstituted".

In connection with aryl radical, cycloalkyl radical, or heterocyclyl radical, "condensed with" is understood as meaning that the ring-system of the aryl radical, the cycloalkyl radical, or the heterocyclyl radical is sharing two atoms (one) of its ring(s) with a ring of the mono- or polycyclic ring-system it is condensed with.

Aliphatic radicals/groups, as referred to in the present invention, are optionally mono- or polysubstituted and may be branched or linear, saturated or unsaturated. Aliphatic radicals, as defined in the present invention, include alkyl, alkenyl and alkinyl radicals. Unsaturated aliphatic radicals, as defined in the present invention, include alkenyl and alkinyl radicals. Preferred aliphatic radicals according to the present invention include but are not restricted to methyl, ethyl, vinyl (ethenyl), ethinyl, propyl, n-propyl, isopropyl, allyl (2-propenyl), 1-propinyl, methylethyl, butyl, n-butyl, iso-butyl, sec-butyl, tert-butyl butenyl, butinyl, 1-methylpropyl, 2-methylpropyl, 1,1-dimethylethyl, pentyl, n-pentyl, 1,1-dimethylpropyl, 1,2-dimethylpropyl, 2,2-dimethylpropyl, hexyl, 1-methylpentyl, n-heptyl, n-octyl, n-nonyl and n-decyl.

In the context of this invention, "alkyl", "alkyl radical" or group is understood as meaning saturated, linear or branched hydrocarbons, which can be unsubstituted or mono- or polysubstituted. Thus unsaturated alkyl is understood to encompass alkenyl and alkinyl groups, like e.g. -CH=CH-CH₃ or -C≡C-CH₃, while saturated alkyl encompasses e.g. -CH₃ and -CH₂-CH₃- In these radicals, C₁₋₂-alkyl represents C₁- or C₂-alkyl, C₁₋₃-alkyl represents C₁-, C₂- or C₃-alkyl, C₁₋₄-alkyl represents C₁-, C₂-, C₃- or C₄-alkyl, C₁₋₅-alkyl represents C₁-, C₂-, C₃-, C₄-, or C₅-alkyl, C₁₋₆-alkyl represents C₁-, C₂-, C₃-, C₄-, C₅- or C₆-alkyl, C₁₋₇-alkyl represents C₁-, C₂-, C₃-, C₄-, C₅-, C₆- or C₇-alkyl, C₁₋₈-alkyl represents C₁-, C₂-, C₃-, C₄-, C₅-, C₆-, C₇- or C₈-alkyl, C₁₋₁₀-alkyl represents C₁-, C₂-, C₃-, C₄-, C₅-, C₆-, C₇-, C₈-, C₉- or C₁₀-alkyl and C₁₋₁₈-alkyl represents C₁-, C₂-, C₃-, C₄-, C₅-, C₆-, C₇-, C₈-, C₉-, C₁₀-, C₁₁-, C₁₂-, C₁₃-, C₁₄-, C₁₅-, C₁₆-, C₁₇- or C₁₈-alkyl. The alkyl radicals are preferably methyl, ethyl, vinyl (ethenyl), propyl, allyl (2-propenyl), 1-propinyl, methylethyl, butyl, 1-methylpropyl, 2-methylpropyl, 1,1-dimethylethyl, pentyl, 1,1-dimethylpropyl, 1,2-dimethylpropyl, 2,2-dimethylpropyl, hexyl, 1-methylpentyl, if substituted also CHF₂, CF₃ or CH₂OH etc.

In connection with alkylene, alkyl or aliphatic radical or group - unless defined otherwise - the term "substituted" in the context of this invention is understood as meaning replacement of at least one hydrogen radical by F, Cl, Br, I, NH₂, SH or OH; within that "monosubstituted" means the substitution of exactly one hydrogen radical, whereas "polysubstituted" means the substitution of more than one hydrogen radical with "polysubstituted"radicals being understood as meaning that the replacement takes effect both on different and on the same atoms several times with the same or different substituents, for example three times on the same C atom, as in the case of CF₃, or at different places, as in the case of e.g. -CH(OH)-CH=CH-CHCl₂. Therefore, "optionally at least monsubstituted" means either "not substituted" if the option is not fulfilled, "monosubstituted" or "polysubstituted".

The term "alkylene" is understood as meaning a divalent alkyl group like -CH₂- or -CH₂-CH₂-, with (CH₂)₃₋₆ being understood as meaning -CH₂-CH₂-CH₂-, -CH₂-CH₂-CH₂-CH₂-, -CH₂-CH₂-CH₂-CH₂-CH₂- and -CH₂-CH₂-CH₂-CH₂-CH₂-CH₂-, (CH₂)₁₋₄ is to be understood as meaning - CH₂-, -CH₂-CH₂-, -CH₂-CH₂-CH₂- and -CH₂-CH₂-CH₂-CH₂-, (CH₂)₄₋₅ is to be understood as meaning -CH₂-CH₂-CH₂-CH₂- and -CH₂-CH₂-CH₂-CH₂-CH₂-, etc. An "alkylene" may also be unsaturated.

The term "salt" is to be understood as meaning any form of the active compound used according to the invention in which it assumes an ionic form or is charged and is coupled with a counter-ion (a cation or anion) or is in solution. By this are also to be understood complexes of the active compound with other molecules and ions, in particular complexes which are complexed via ionic interactions.

The term "physiologically acceptable salt" means in the context of this invention any salt that is physiologically tolerated (most of the time meaning not being toxic-especially not caused by the counter-ion) if used appropriately for a treatment especially if used on or applied to humans and/or mammals.

These physiologically acceptable salts can be formed with cations or bases and in the context of this invention is understood as meaning salts of at least one of the compounds used according to the invention - usually a (deprotonated) acid - as an anion with at least one, preferably inorganic, cation which is physiologically tolerated - especially if used on humans and/or mammals. The salts of the alkali metals and alkaline earth metals are particularly preferred, and also those with NH4, but in particular (mono)- or (di)sodium, (mono)- or (di)potassium, magnesium or calcium salts.

These physiologically acceptable salts can also be formed with anions or acids in the context of this invention is understood as meaning salts of at least one of the compounds used according to the invention - usually protonated, for example on the nitrogen - as the cation with at least one anion which are physiologically tolerated - especially if used on humans and/or mammals. By this is understood in particular, in the context of this invention, the salt formed with a physiologically tolerated acid, that is to say salts of the particular active compound with inorganic or organic acids which are physiologically tolerated - especially if used on humans and/or mammals. Examples of physiologically tolerated salts of particular acids are salts of: hydrochloric acid, hydrobromic acid, sulfuric acid, methanesulfonic acid, formic acid, acetic acid, oxalic acid, succinic acid, malic acid, tartaric acid, mandelic acid, fumaric acid, lactic acid or citric acid.

The compounds of the invention may be in crystalline form or either as free compounds or as solvates and it is intended that those forms are within the scope of the present invention. Methods of solvation are generally known within the art. Suitable solvates are pharmaceutically acceptable solvates. The term "solvate" according to this invention is to be understood as meaning any form of the active compound according to the invention in which this compound has attached to it via non-covalent binding another molecule (most likely a polar solvent) especially including hydrates and alcoholates, e.g. methanolate.

Unless otherwise stated, the compounds of the invention are also meant to include compounds which differ only in the presence of one or more isotopically enriched atoms. For example, compounds having the present structures except for the replacement of a hydrogen by a deuterium or tritium, or the replacement of a carbon by ¹³C- or ¹⁴C-enriched carbon or ¹⁵N-enriched nitrogen are within the scope of this invention.

Any compound that is a prodrug of a compound of formula (I) is within the scope of the invention. The term "prodrug" is used in its broadest sense and encompasses those derivatives that are converted in vivo to the compounds of the invention. Such derivatives would readily occur to those skilled in the art, and include, depending on the functional groups present in the molecule and without limitation, the following derivatives of the present compounds: esters, amino acid esters, phosphate esters, metal salts sulfonate esters, carbamates, and amides. Examples of well known methods of producing a prodrug of a given acting compound are known to those skilled in the art and can be found e.g. in Krogsgaard-Larsen et al. "Textbook of Drug design and Discovery" Taylor & Francis (April 2002).

The compounds of formula (I) or their salts or solvates are preferably in pharmaceutically acceptable or substantially pure form. By pharmaceutically acceptable form is meant, inter alia, having a pharmaceutically acceptable level of purity excluding normal pharmaceutical additives such as diluents and carriers, and including no material considered toxic at normal dosage levels. Purity levels for the drug substance are preferably above 50%, more preferably above 70%, most preferably above 90%. In a preferred embodiment it is above 95% of the compound of formula (I) or, or of its salts, solvates or prodrugs.

Particularly preferred are compounds according to the invention which are compounds of general formula (Ia), 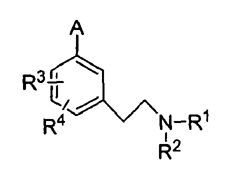 , wherein
A is a compound selected from the following group 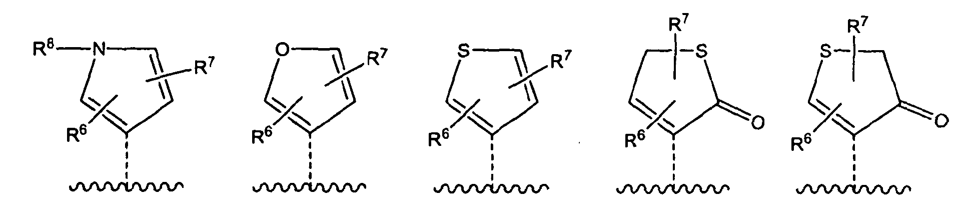 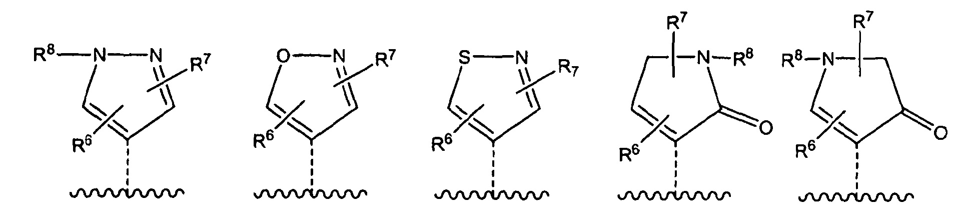 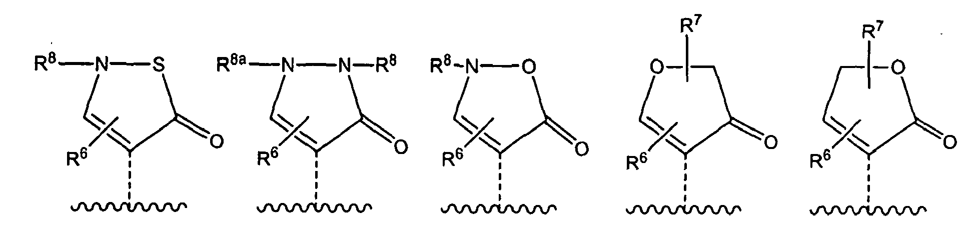 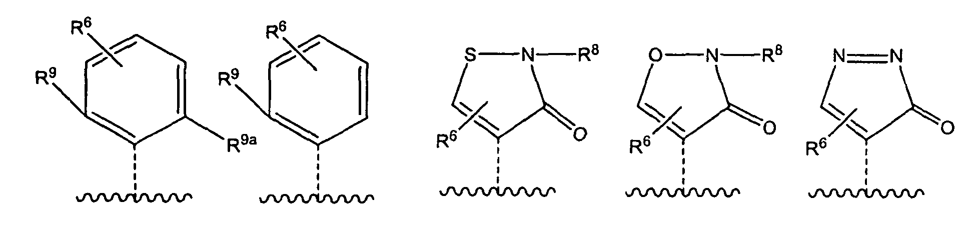
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH.

In a preferred embodiment the following proviso(s)/disclaimer(s) applies:
- with the proviso that
   if R¹ and R² are both CH₃, R³ and R⁴ are both H, A is 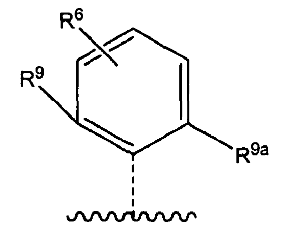 and one of R⁹ or R^{9a} is -CH=CH₂, the other may not be OCH₃;
   and/or
- with the proviso that
   if R¹ and R² are both H, one of R³ and R⁴ is H, while the other is -O(C₂H₅), A is 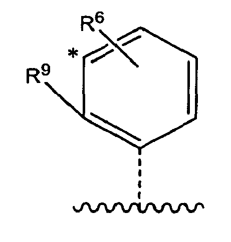 and R⁹ is -OCH₃, R⁶ may not be OCH₃ in the position marked with "*".

Also particularly preferred is a compound according to the invention, which is a compound according to Formula Ia, wherein
A is a compound selected from the following group 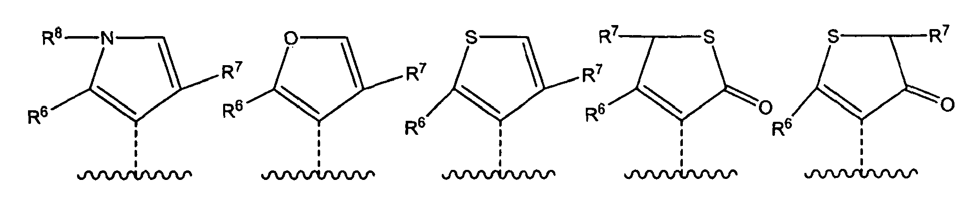 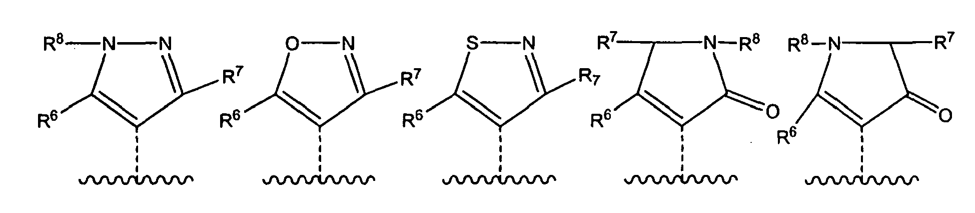 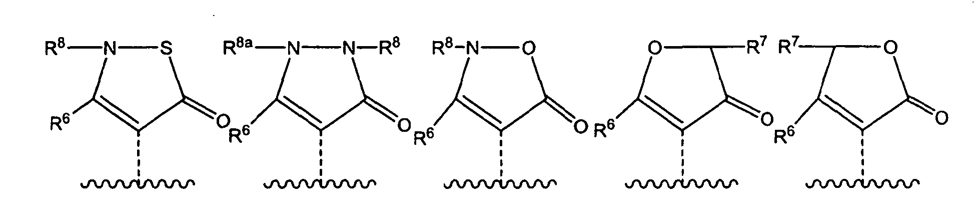 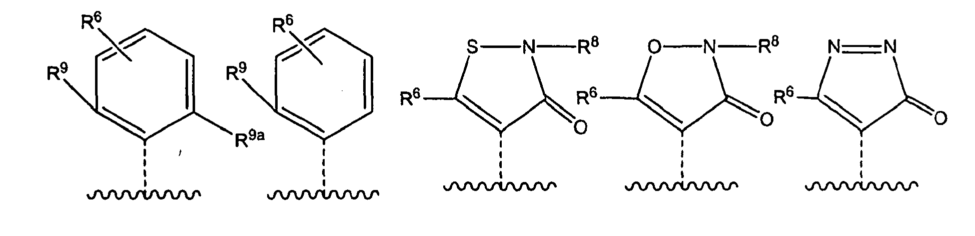
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH.

In a preferred embodiment the following proviso(s)/disclaimer(s) applies:
- with the proviso that
   if R¹and R² are both CH₃, R³ and R⁴ are both H, A is 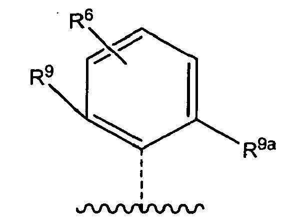 and one of R⁹ or R^{9a} is -CH=CH₂, the other may not be OCH₃;
   and/or
- with the proviso that
   if R¹ and R² are both H, one of R³ and R⁴ is H, while the other is -O(C₂H₅), A is 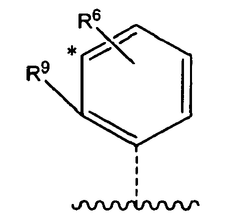 and R⁹ is -OCH₃, R⁶ may not be OCH₃ in the position marked with "*".

Also particularly preferred is a compound according to the invention, which is a compound according to Formula Ia, wherein
A is a compound selected from the following group 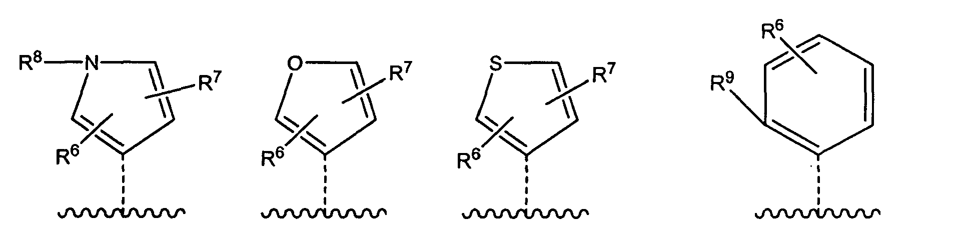 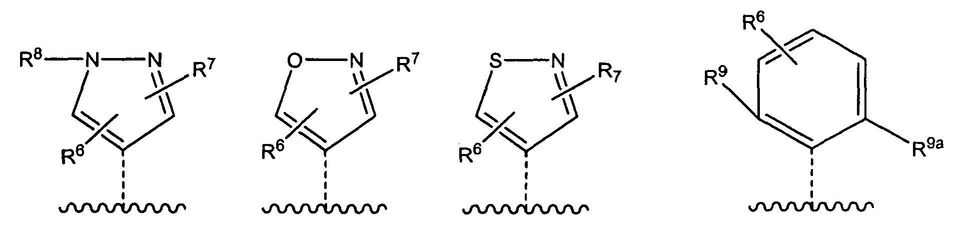 or 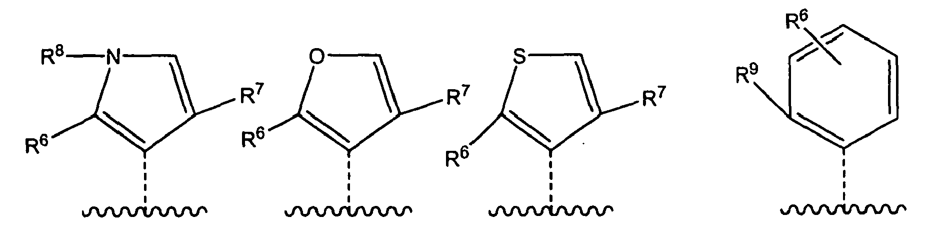 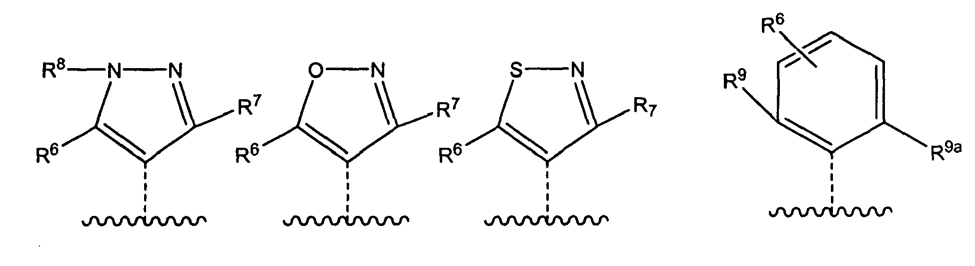
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;

In a preferred embodiment the following proviso(s)/disclaimer(s) applies:
- with the proviso that
   if R¹ and R² are both CH₃, R³ and R⁴ are both H, A is 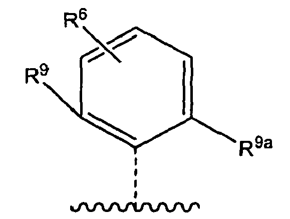 and one of R⁹ or R^{9a} is -CH=CH₂, the other may not be OCH₃;
   and/or
- with the proviso that
   if R¹ and R² are both H, one of R³ and R⁴ is H, while the other is -O(C₂H₅), A is 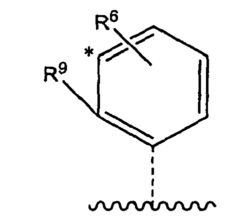 and R⁹ is -OCH₃, R⁶ may not be OCH₃ in the position marked with "*".

Also particularly preferred is a compound according to the invention, which is a compound of GROUP A according to Formula Ia, wherein
A is a compound selected from the following group 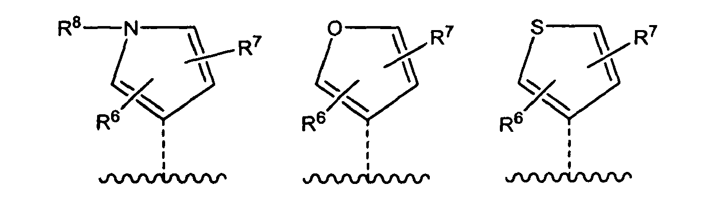 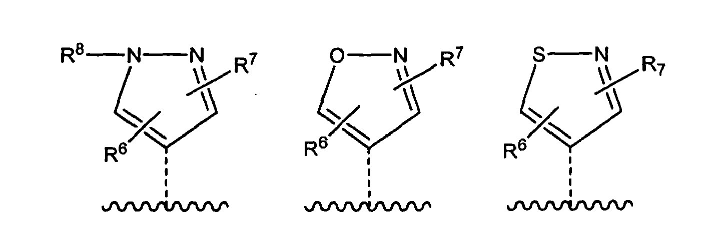 or 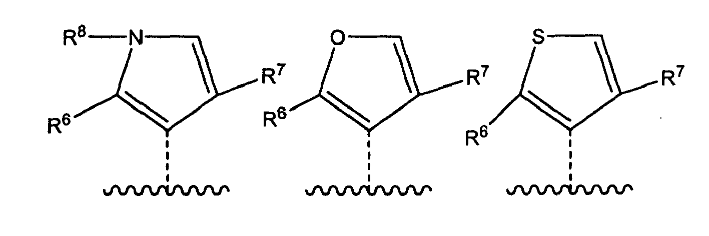 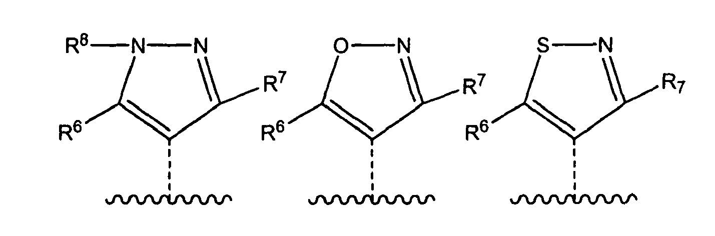
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem;
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ is selected from hydrogen; or an aliphatic radical, which is linear or branched, saturated or unsaturated, and optionally at least mono-substituted by F, Cl, Br, I, SH or OH.

Also particularly preferred is a compound according to the invention, which is a compound of GROUP B according to Formula la, wherein
A is a compound selected from the following group 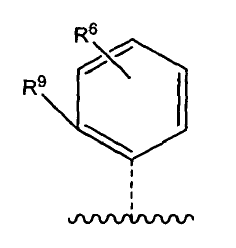 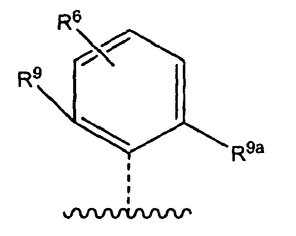
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ is selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH.

In a preferred embodiment the following proviso(s)/disclaimer(s) applies for GROUP B:
- with the proviso that
   if R¹ and R² are both CH₃, R³ and R⁴ are both H, A is 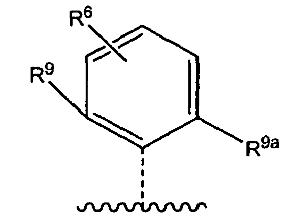 and one of R⁹ or R^{9a} is -CH=CH₂, the other may not be OCH₃;
   and/or
- with the proviso that
   if R¹ and R² are both H, one of R³ and R⁴ is H, while the other is -O(C₂H₅), A is 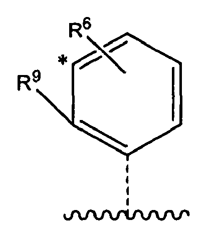 and R⁹ is -OCH₃, R⁶ may not be OCH₃ in the position marked with "*".

Also particularly preferred is a compound according to the invention, which is a compound of GROUP A according to Formula Ia, wherein
R¹ and R² each are independently selected from the group consisting of hydrogen; or a linear or branched, optionally at least mono-substituted C₁₋₄-alkyl radical; or
R¹ and R² together with the bridging nitrogen atom form an saturated or unsaturated, optionally at least mono-substituted 5- or 6-membered-heterocyclic ring;
preferably in that
R¹ and R² are each independently selected from the group consisting of hydrogen; or a linear or branched C₁₋₄-alkyl radical; or
R¹ and R² together with the bridging nitrogen atom form an saturated or unsaturated, optionally at least mono-substituted 5- or 6-membered-heterocyclic ring;
more preferably in that
R¹ and R² are each independently selected from the group consisting of hydrogen, CH₃, C₂H₅ or C₃H₇.

Also particularly preferred is a compound according to the invention, which is a compound of GROUP A according to Formula Ia, wherein
R³ and R⁴ are independently from each other selected from hydrogen; halogen, OH, SH, NH₂; a linear or branched, optionally at least mono-substituted C₁₋₄-alkyl radical; or O-R with R being a linear or branched, optionally at least mono-substituted C₁₋₄-alkyl radical;
preferably in that
R³ and R⁴ are independently from each other selected from H, F, Cl, Br, I, OH, SH, NH₂, CH₃, C₂H₅, C₃H₇, C₄H₉, OCH₃, OC₂H₅, OC₃H₇ or OC₄H₉,
more preferably in that
R³ and R⁴ are H.

Also particularly preferred is a compound according to the invention, which is a compound of GROUP A according to Formula la, wherein
R⁶ and R⁷ are independently from each other selected from hydrogen; halogen, OH, SH, NH₂; a C₁₋₄-alkyl radical, which is linear or branched, and optionally at least mono-substituted by F, Cl, Br, I, SH or OH; or O-R with R being a C₁₋₄-alkyl radical, which is linear or branched, and optionally at least mono-substituted by F, Cl, Br, I, SH or OH;
preferably in that
R⁶ and R⁷ are independently from each other selected from H, F, Cl, Br, I, OH, SH, NH₂, CH₃, C₂H₅, C₃H₇, C₄H₉, OCH₃, OC₂H₅, OC₃H₇ or OC₄H₉;
more preferably in that
R⁶ and R⁷ are independently from each other selected from H, or CH₃.

Also particularly preferred is a compound according to the invention, which is a compound of GROUP A according to Formula Ia, wherein
R⁸ is selected from hydrogen; or a C₁₋₄-alkyl radical, which is linear or branched, and optionally at least mono-substituted by F, Cl, Br, I, SH or OH;
preferably in that
R⁸ is selected from H, F, Cl, Br, I, OH, SH, NH₂, CH₃, C₂H₅, C₃H₇, or C₄H₉;
more preferably in that
R⁸ is selected from H or CH₃.

Also particularly preferred is a compound according to the invention, which is a compound of GROUP A according to Formula la, selected from
- Dimethyl-{2-[3-(1,3,5-trimethyl-1H-pyrazol-4-yl)-phenyl]-ethyl}-amine,
- Methyl-{2-[3-(1,3,5-trimethyl-1H-pyrazol-4-yl)-phenyl]-ethyl}-amine,
- Diethyl-{2-[3-(1,3,5-trimethyl-1H-pyrazol-4-yl)-phenyl]-ethyl}-amine,
- Dipropyl-{2-[3-(1,3,5-trimethyl-1H-pyrazol-4-yl)-phenyl]-ethyl}-amine,
- {2-[3-(3,5-Dimethyl-1 H-pyrazol-4-yl)-phenyl]-ethyl}-dimethyl-amine,
- {2-[3-(3,5-Dimethyl-1H-pyrazol-4-yl)-phenyl]-ethyl}-methyl-amine,
- {2-[3-(3,5-Dimethyl-1H-pyrazol-4-yl)-phenyl]-ethyl}-diethyl-amine,
- {2-[3-(3,5-Dimethyl-1H-pyrazol-4-yl)-phenyl]-ethyl}-dipropyl-amine,
- Dimethyl-{2-[3-(1-methyl-1H-pyrazol-4-yl)-phenyl]-ethyl}-amine,
- Methyl-{2-[3-(1-methyl-1H-pyrazol-4-yl)-phenyl]-ethyl}-amine,
- Diethyl-{2-[3-(1-methyl-1H-pyrazol-4-yl)-phenyl]-ethyl}-amine,
- {2-[3-(1-Methyl-1H-pyrazol-4-yl)-phenyl]-ethyl}-dipropyl-amine,
- {2-[3-(3,5-Dimethyl-isoxazol-4-yl)-phenyl]-ethyl}-dimethyl-amine,
- {2-[3-(3,5-Dimethyl-isoxazol-4-yl)-phenyl]-ethyl}-methyl-amine,
- {2-[3-(3,5-Dimethyl-isoxazol-4-yl)-phenyl]-ethyl}-diethyl-amine, or
- {2-[3-(3,5-Dimethyl-isoxazol-4-yl)-phenyl]-ethyl}-dipropyl-amine,
optionally in form of a salt, preferably a physiologically acceptable salt, more preferably in form of a physiologically acceptable acid addition salt, most preferably a hydrochloride salt, or a corresponding solvate.

Also particularly preferred is a compound according to the invention, which is a compound of GROUP B according to Formula Ia, wherein
R⁶ is selected from hydrogen; halogen, OH, SH, NH₂; a C₁₋₄-alkyl radical, which is linear or branched, and optionally at least mono-substituted by F, Cl, Br, I, SH or OH; or O-R with R being a C₁₋₄-alkyl radical, which is linear or branched, and optionally at least mono-substituted by F, Cl, Br, I, SH or OH;
preferably in that
R⁶ is selected from H, F, Cl, Br, I, OH, SH, NH₂, CH₃, C₂H₅, C₃H₇, C₄H₉, OCH₃, OC₂H₅, OC₃H₇ or OC₄H₉;
more preferably in that
R⁶ is H.

Also particularly preferred is a compound according to the invention, which is a compound of GROUP B according to Formula Ia, wherein
R⁹ and R^{9a} are independently from each other selected from a C₁₋₄-alkyl radical, which is linear or branched, and optionally at least mono-substituted by F, Cl, Br, I, SH or OH; or O-R with R being a C₁₋₄-alkyl radical, which is linear or branched, and optionally at least mono-substituted by F, Cl, Br, I, SH or OH;
preferably in that
R⁹ and R^{9a} are independently from each other selected from CH₃, C₂H₅, C₃H₇, C₄H₉, OCH₃, OC₂H₅, OC₃H₇ or OC₄H₉;
more preferably in that
R⁹ and R^{9a} are independently from each other selected from CH₃, or OCH₃.
most preferably in that
R⁹ and R^{9a} are both selected either from CH₃, or OCH₃.

Also particularly preferred is a compound according to the invention, which is a compound of GROUP B according to Formula la selected from
- [2-(2',6'-Dimethyl-biphenyl-3-yl)-ethyl]-dimethyl-amine,
- [2-(2',6'-Dimethyl-biphenyl-3-yl)-ethyl]-methyl-amine,
- [2-(2',6'-Dimethyl-biphenyl-3-yl)-ethyl]-diethyl-amine,
- [2-(2',6'-Dimethyl-biphenyl-3-yl)-ethyl]-dipropyl-amine,
- [2-(2',6'-Dimethoxy-biphenyl-3-yl)-ethyl]-dimethyl-amine,
- [2-(2',6'-Dimethoxy-biphenyl-3-yl)-ethyl]-methyl-amine,
- [2-(2',6'-Dimethoxy-biphenyl-3-yl)-ethyl]-diethyl-amine,
- [2-(2',6'-Dimethoxy-biphenyl-3-yl)-ethyl]-dipropyl-amine,
- [2-(2'-Methoxy-biphenyl-3-yl)-ethyl]-dimethyl-amine,
- [2-(2'-Methoxy-biphenyl-3-yl)-ethyl]-methyl-amine,
- Diethyl-[2-(2'-methoxy-biphenyl-3-yl)-ethyl]-amine, or
- [2-(2'-Methoxy-biphenyl-3-yl)-ethyl]-dipropyl-amine,
optionally in form of a salt, preferably a physiologically acceptable salt, more preferably in form of a physiologically acceptable acid addition salt, most preferably a hydrochloride salt, or a corresponding solvate.

In a further aspect the present invention also provides a process for the preparation of compounds of general formula (I), wherein a compound of general formula (VI) 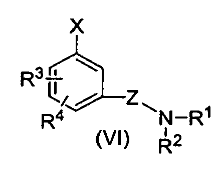 , wherein R¹, R², R³, R⁴ and Z are as defined in claim 1, and X represents halogen, preferably Br, or an O-triflate group, is reacted with a compound of general formula VII or Vlla 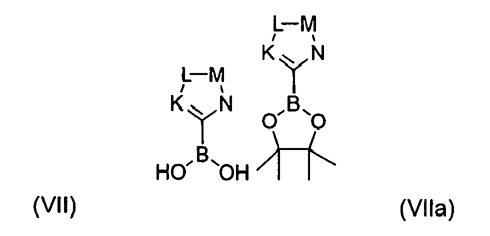 , wherein K, L, M, and N are as defined in claim 1, to form a compound according to formula I, preferably in presence of a catalyst.

In a preferred embodiment of this process
a) the catalyst is a palladium catalyst with or without a ligand, and/or
b) the reaction is carried out in presence of at least one base, selected from organic or inorganic bases and/or
c) the reaction is carried out in a suitable reaction medium selected from ethers, alcohols, hydrocarbons or other organic solvents.

In a closely related aspect of the present invention is also provided a process for the preparation of compounds of general formula (I), wherein R₁, R₂, R₃, R₄, K, L, M, N and Z have the meaning given above, according to which at least one compound of general formula (VII) or (VIIa), (Scheme 1) 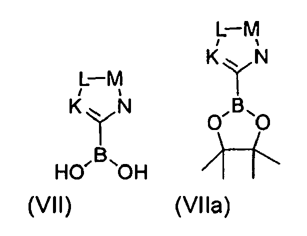 wherein K, L, M and N have the meaning given above, is subjected to cross-coupling Suzuki reaction with at least one compound of general formula (VI), 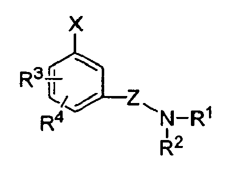 wherein R₁, R₂, R₃, R₄, and Z have the meaning given above and X represent halogen, preferably bromide; or O-triflate group, in a suitable reaction medium, in the presence of a palladium catalyst, a suitable ligand and at least one base. This process can be performed by subjecting the reaction mixture to 100°C by conventional heating for 20h, or by microwave radiation for a period of time sufficient to achieve the title compound (I), preferably for 1 to 10 minutes, and at a temperature between 100 to 120°C.

The compounds of general formulas (VII) and (VIIa) are either commercially available or can be produced according to methods known to those skilled in the art.

Suitable reaction media are e.g. organic solvents, such as ethers, preferably diethyl ether, dioxane, tetrahydrofurane, dimethyl glycol ether, or alcohols, e.g. methanol, ethanol, propanol, isopropanol, butanol, isobutanol, tert-butanol, or hydrocarbons, preferably benzene, toluene, xylene, hexane, cyclohexane, petroleum ether, or halogenated hydrocarbons, e.g. dichloromethane, trichloromethane, tetrachloromethane, dichloroethylene, trichloroethylene, chlorobenzene or/and other solvents preferably ethyl acetate, triethylamine, pyridine, dimethulsulfoxide, dimethylformamide, hexamethylphosphoramide, acetonitrile, acetone or nitromethane are included. Mixtures based one or more of the above mentioned solvents and water may also be used.

According to the invention, the bases that may be used in the process are generally organic or inorganic bases, preferably alkali metal hydroxides, e.g. sodium hydroxide or potassium hydroxide, or obtained from other metals such as barium hydroxide or different carbonates, preferably potassium carbonate, sodium carbonate, calcium carbonate or alkoxydes, e.g. sodium methoxide potassium methoxide, sodium ethoxide, potassium ethoxide or potassium tert-butoxide, or organic amines, preferably triethylamine, diisopropylethylamine or heterocycles, e.g. 1,4-diazabicyclo[2.2.2]octane, 1,8-diazabicyclo5.4.0]undec-7-ene, pyridine, diamino pydine, dimethylaminopyridine, methylpiperidine or morpholine. Alkali metals such as sodium or its hydrides, e.g. sodium hydride, may also be used.

Preparation of compounds of general formula (VI) can be achieved by reductive amination reaction of aldehydes of general formula (VIII), 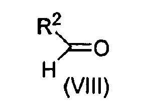 wherein R₂ have the meaning given above, with a compound of general formula (IX), 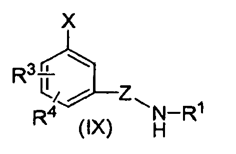 wherein R₁, R₃, R₄, X and Z have the meaning given above. The reductive amination is performed by subjecting a reaction mixture comprising a compound of general formula (VIII), and amino compound of general formula (IX) and a reducing agent in a reaction medium, to microwave radiation for a period of time sufficient to achieve the title compound (VI), preferably for 1 to 10 minutes, and at a temperature between 90 to 120°C. The use of microwave irradiation limits the formation of undesirable secondary reaction products, compared to what is obtained in a conventional reductive amination procedure.

This process can be performed as a direct reaction when the carbonyl compound of general formula (VIII) and the amine compound of general formula (IX) are mixed with the reducing agent without prior formation of the intermediate imine or iminium salt. A stepwise or indirect reaction involves the reduction of the preformatted imine in a separate step.

The choice of the reducing agent can be conventionally made by those skilled in the art. Reducing agents useful in this procedure include hydrogen and a catalyst, zinc and HCl, sodium cyanoborohydride, lithium cyanoborohydride, tetrabutylammonium cyanoborohydride, cyanoborohydride on a solid support, sodium cyanoborohydride and dehydrating agents, sodium cyanoborohydride and titanium additives, sodium cyanoborohydride and zinc halide additives, sodium borohydride, sodium borohydride and dehydrating agents, sodium borohydride and titanium additives, sodium borohydride and zinc salt additives, lithium borohydride, potassium borohydride, polymer-supported borohydride, borohydride exchange resin with nickel acetate or palladium acetate, sodium triacetoxyborohydride, sodium triacetoxyborohydride and additives, tetramethylammonium triacetoxyborohydride, sodium cyano-9-borabicyclo[3.3.1]nonane, lithium triethylborohydride, lithium tri(*sec*-butyl)borohydride, sodium diisopinocampheylcyanoborohydride, amine boranes, borane-pyridine complex and alkylamine boranes. Sodium triacetoxyborohydride is particularly preferred because is non-toxic and generally does not reduce the carbonyl group prior to imine formation.

Suitable reaction media are those described above.

Preparation of compounds of general formula (IX) can be achieved by reductive amination reaction of aldehydes of general formula (X), 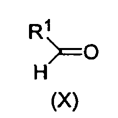 wherein R₁ have the meaning given above, with a compound of general formula (XI), 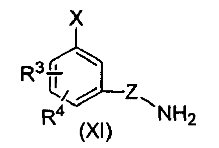 wherein R₃, R₄, X and Z have the meaning given above. The reductive amination is performed in the same conditions described above.

In the case that R₁ and R₂ have the same meaning, the preparation of compound with general formula (VI) can be achieved by direct reductive amination reaction of at least 2 equivalents of the corresponding aldehyde and compound with general formula (XI).

The compounds of general formulas (VIII) and (X) are either commercially available or can be produced according to methods known to those skilled in the art.

The preparation of compounds of general formula (I) is illustrated in scheme 1:

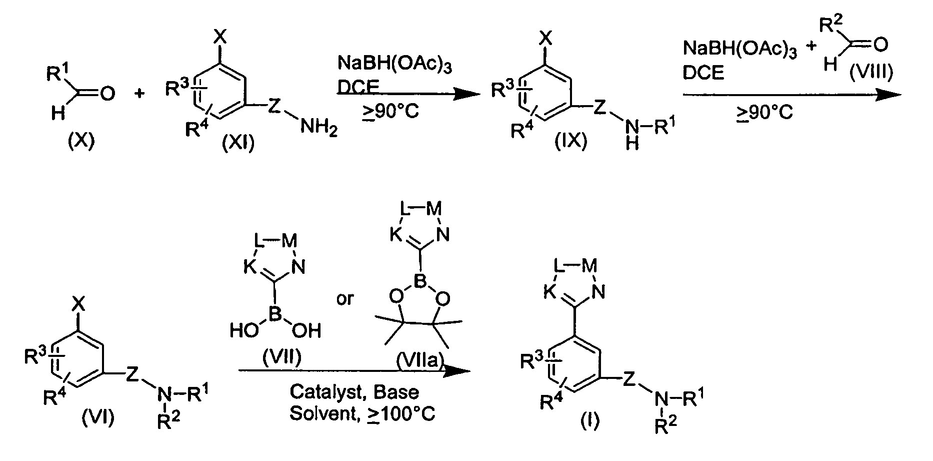

In another aspect, the present invention also provides a process for the preparation of compounds of general formula (la), according to Scheme 2, wherein R₁, R₂, R₃, R₄, and A have the meaning given above.

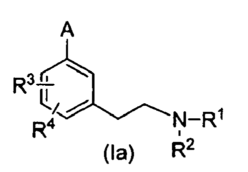

The compounds of general formula (Ia) can be prepared by cross-coupling Suzuki reaction of boronic acids or boronate esters of general formula (XII) or (XIIa), 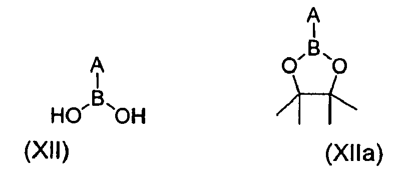 wherein A have the meaning given above, with at least one compound of general formula (XIII), 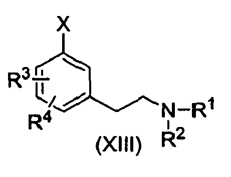 wherein R₁, R₂, R₃ and R₄, have the meaning given above and X represent halogen, preferably bromide or *O*-triflate group, in a suitable reaction medium, in the presence of a palladium catalyst, a suitable ligand and at least one base. This process can be performed by subjecting the reaction mixture to 100°C by conventional heating for 20h, or by microwave radiation for a period of time sufficient to achieve the title compound (I), preferably for 1 to 10 minutes, and at a temperature between 100 to 120°C.

The compounds of general formulas (XII) and (XIIa) are either commercially available or can be produced according to methods known to those skilled in the art.

Suitable reaction media are those described above.

The bases that may be used in the process are those descried above.

Preparation of compounds of general formula (XIII) can be achieved by reductive amination reaction of aldehydes of general formula (VIII), 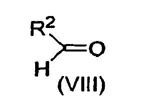 wherein R₂ have the meaning given above, with a compound of general formula (XIV), 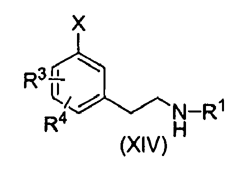 wherein R₁, R₃, X and R₄ have the meaning given above. The reductive amination is performed by subjecting a reaction mixture comprising a compound of general formula (VIII), and amino compound of general formula (XIV) and a reducing agent in a reaction medium, to microwave radiation for a period of time sufficient to achieve the title compound (XIII), preferably for 1 to 10 minutes, and at a temperature between 90 to 120°C. The use of microwave irradiation limits the formation of undesirable secondary reaction products, compared to what is obtained in a conventional reductive amination procedure.

This process can be performed as a direct reaction when the carbonyl compound of general formula (VIII) and the amine compound of general formula (XIV) are mixed with the reducing agent without prior formation of the intermediate imine or iminium salt. A stepwise or indirect reaction involves the reduction of the preformatted imine in a separate step.

The choice of the reducing agent can be conventionally made by those skilled in the art. Reducing agents useful in this procedure are those described above.

Suitable reaction media are those described above.

Preparation of compounds of general formula (XIV) can be achieved by reductive amination reaction of aldehydes of general formula (X), 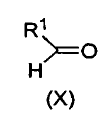 wherein R₁ have the meaning given above, with a compound of general formula (XV), 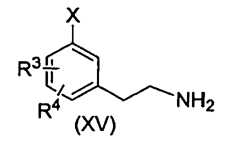 wherein R₃ and R₄ and X have the meaning given above. The reductive amination is performed in the same conditions described above.

In the case that R₁ and R₂ have the same meaning, the preparation of compound with general formula (XIII) can be achieved by direct reductive amination reaction of at least 2 equivalents of the corresponding aldehyde and compound with general formula (XV).

The preparation of compounds of general formula (Ia) is illustrated in scheme 2:

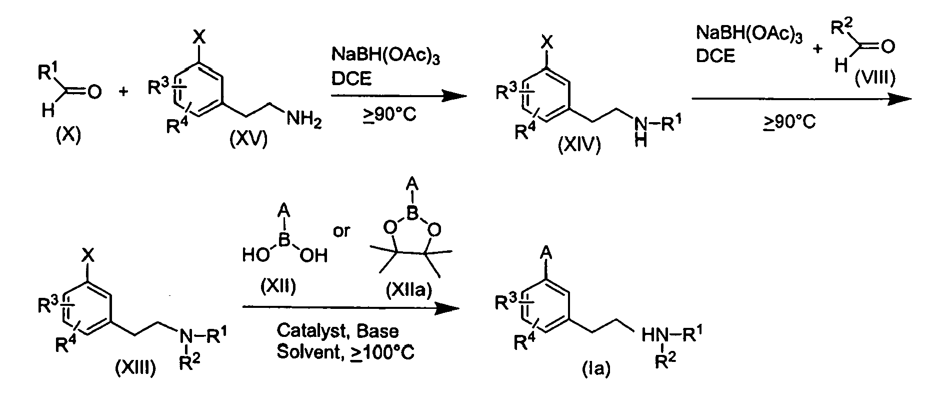

In another aspect, the present invention also provides an alternative process for the preparation of compounds of general formula (Ia), according to Scheme 3, wherein R₁, R₂, R₃, and R₄, have the meaning given above and A is: 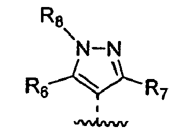 wherein R₆, R₇ and R₈ have the meaning described above. This a very suitable process, also when R³ and R⁴ are both hydrogen.

The compounds of general formula (XVI), 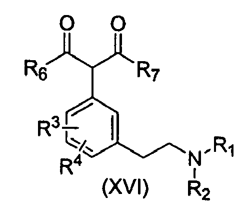 wherein R₁, R₂, R₆ and R₇ have the meaning described above were reacted with compounds of general formula (XVII), 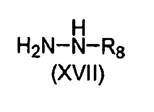 wherein R₈ have the meaning described above, in a suitable reaction media to give the title compounds of general formula la.

Preparation of compounds of general formula (XVI) can be achieved by Cu catalyzed nucleophilic substitution reaction of compounds of general formula (XVIII), 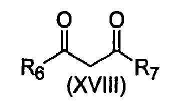 wherein R₆ and R₇ have the meaning described above, with compounds of general formula (XIII), 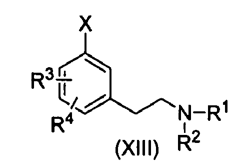 wherein R₁, R₂, R₃ and R₄, have the meaning given above and X represent halogen, preferably iodide or bromide in a suitable reaction medium, in the presence of CuX, and at least one base.

The compounds of general formulas (XVII) and (XVIII) are either commercially available or can be produced according to methods known to those skilled in the art.

Suitable reaction media are those described above.

The bases that may be used in the process are those descried above.

This alternative preparation of compounds of general formula (Ia) is illustrated in scheme 3:

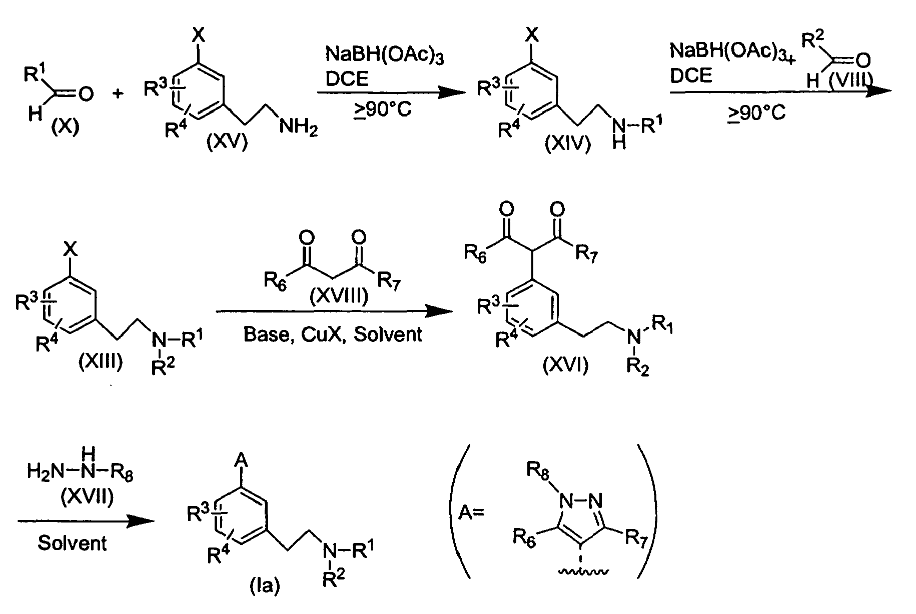

In a further aspect the present invention also provides a process for the preparation of salts of compounds of general formula (I), wherein at least one compound of general formula (I) is reacted with an inorganic and/or organic acid, preferably in the presence of a suitable reaction medium. Suitable reaction media are the ones given above. Suitable inorganic acid are for example hydrochloric acid, hydrobromic acid, phosphoric acid, sulphuric acid, nitric acid. Suitable organic acids are e.g. citric acid, maleic acid, furmaric acid, tartaric acid or derivatives thereof, such as p-toluenesulfonic acid, methanesulfonic acid or camphersulfonic acid.

In yet a further aspect the present invention also provides a process for the preparation of salts of compounds of general formula (I), wherein at least one compound of general formula (I) having at least one acidic group is reacted with one or more suitable bases, preferably in the presence of suitable reaction medium. Suitable bases are e.g. hydroxides. Carbonates or alkoxides, which include suitable cations, derived e.g. from alkaline metals, alkaline earth metals or organic cations, e.g. [NHₙR₄₋ₙ]⁺, wherein n is 0, 1, 2, 3 or 4 and R represents a branched or linear C₁₋₄ alkyl radical.

Solvates, preferably hydrates, of the phenylamino-substituted piperidine compounds of general formula (I), or corresponding stereoisomers, or corresponding salts may also be obtained by standard procedures known to those skilled in the art.

If the compounds of general formula (I) are obtained in form of a mixture of stereoisomers, particularly enantiomers or diastereomers, said mixtures may be separated by standard procedures known to those skilled in the art, e.g. chromatographic methods of crystallization with chiral reagents.

The purification and isolation of the phenylamino-substituted piperidine compounds of general formula (I) or a corresponding stereoisomer, or a corresponding salt, or corresponding solvate respectively, if required may be carried out by conventional methods known to those skilled in the art, e.g. chromatographic methods or recrystallization.

The compounds of general formula (I), their stereoisomers or the respective salts or solvates are toxicologically acceptable and are therefore suitable as pharmaceutical active substances for the preparation of medicaments.

The present invention therefore also provides for a pharmaceutical formulation or medicament comprising at least one compound according to formula I, 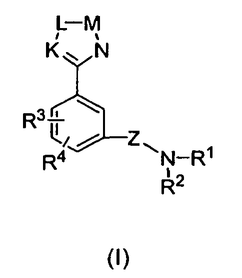 wherein
K-L-M-N together form
- =CH-X-Y=CH-; in which any suitable H may be substituted by R⁶ and/or R⁷, and in which X is selected from NR⁸, O or S, while Y is selected from N or CH;
- =CH-X-Y-C(O)-; in which any suitable H may be substituted by R⁶ and in which one of X and Y is NR⁸, while the other is selected from NR^{8a}, S or O;
- =CH-X-Y-C(O)-; in which one of X and Y is CH₂, while the other is selected from NR⁸, S or O, in which any suitable H may be substituted by R⁶ and/or R⁷;
- =CR⁶-N=N-C(O)-;
- =CR⁹-CH=CH-CH=CH-; in which any suitable H may be substituted by R⁶;
- =CR⁹-CH=CH-CH=CR^{9a}-; in which any suitable H may be substituted by R⁶;
- =CH-X=Y-CH=CH-; in which any suitable H may be substituted by R⁶ and/or R⁷, and in which one of X or Y is selected from N, while the other is selected from N or CH;
- =CH-X=Y-CH₂-CH₂-; in which any suitable H may be substituted by R⁶ and/or R⁷, and in which one of X or Y is selected from N, while the other is selected from N or CH;
- =CH-X-Y-CH=CH-; in which any suitable H may be substituted by R⁶ and/or R⁷, and in which one of X or Y is selected from NR⁸, O or S while the other is selected from NR^{8a} or CH₂;
- =CH-X-Y-CH₂-CH₂-; in which any suitable H may be substituted by R⁶ and/or R⁷, and in which one of X or Y is selected from NR⁸, O or S while the other is selected from NR^{8a} or CH₂;
- =CH-X-CH₂-Y=CH-; in which any suitable H may be substituted by R⁶ and/or R⁷, and in which X is selected from NR⁸, O or S while Y is selected from N or CH;
- =CH-X-CH=Y-CH₂-; in which any suitable H may be substituted by R⁶ and/or R⁷, and in which X is selected from NR⁸, O or S while Y is selected from N or CH;
- =CH-N=CH-Y=CH-; in which any suitable H may be substituted by R⁶ and/or R⁷;
- =CH-X-CH₂-Y-CH₂-; in which any suitable H may be substituted by R⁶ and/or R⁷, and in which one of X or Y is selected from NR⁸, O or S while the other is selected from NR^{8a}, O, S or CH₂;

R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem;
Z is selected from
   - -(CH₂)ₙ-, with n being 1, 2, 3 or 4;
   - -O-(CH₂)ₙ-, with n being 1, 2, 3 or 4;
   - -S-(CH₂)ₙ-, with n being 1, 2, 3 or 4;
   - (CH₂)ₙ-(CHR⁵)-(CH₂)ₘ, with n and m being selected from 0, 1, 2 or 3 and m+n being 1, 2 or 3, with R⁵ being selected from F, Cl, Br, I, OH, SH, or unsubstituted C₁₋₄-Alkyl;
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants.

In a preferred embodiment of the above medicament according to the invention the medicament comprises at least one compound according to formula la, 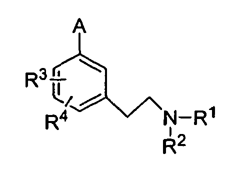 , wherein
A is a compound selected from the following group 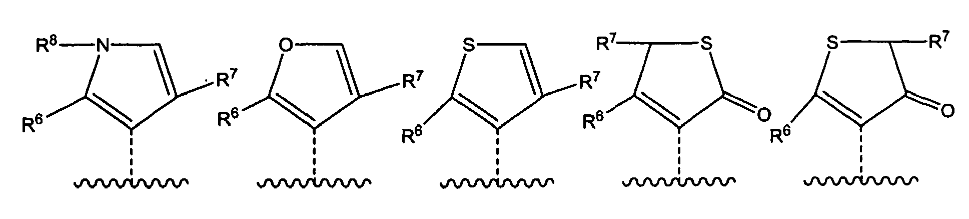 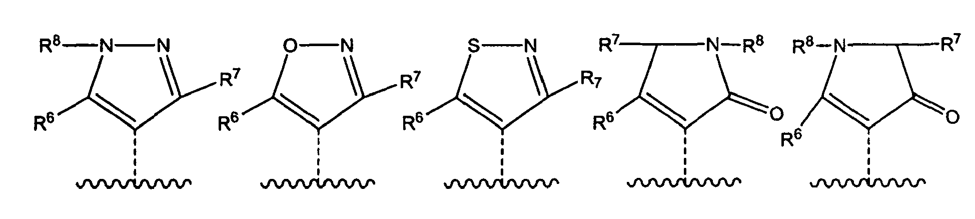 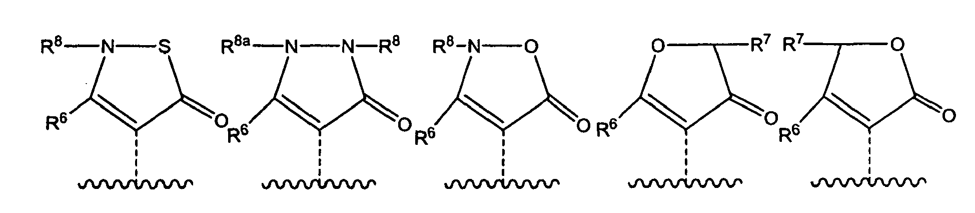 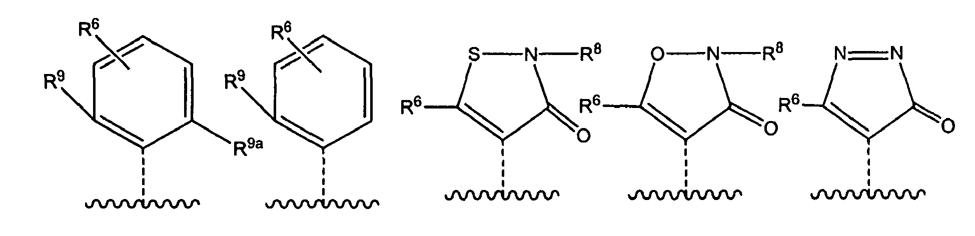
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants.

In another preferred embodiment of the above medicament according to the invention the medicament comprises at least one compound according to formula la, 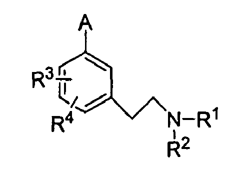 , wherein
A is a compound selected from the following group 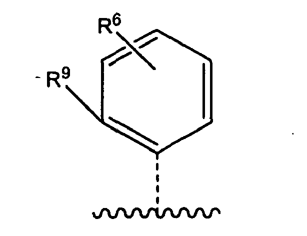 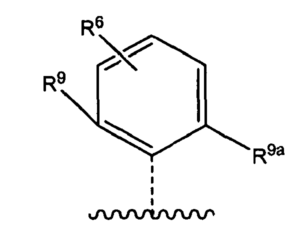
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ is selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants.

Another aspect of the invention is a medicament/pharmaceutical composition comprising at least one compound according to the invention, optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants.

Furthermore, the present invention also provides for a pharmaceutical composition/medicament comprising at least one compound of general formula (I), optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants, which is not yet formulated into a medicament.

Preferably the medicament is suitable for the treatment of a 5-HT₇ mediated disease or condition, especially selected from pain, preferably visceral pain, chronic pain, cancer pain, migraine, acute pain or neuropathic pain, more prefearably neuropathic pain, allodynia or hyperalgesia or selected from sleep disorder, shift worker syndrome, jet lag, depression, seasonal affective disorder, migraine, anxiety, psychosis, schizophrenia, cognition and memory disorders, neuronal degeneration resulting from ischemic events, cardiovascular diseases such as hypertension, irritable bowel syndrome, inflammatory bowel disease, spastic colon or urinary incontinence.

The present invention also provides for the use of at least one compound according to formula I, 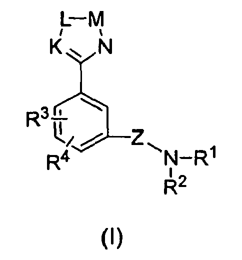 wherein
K-L-M-N together form
- =CH-X-Y=CH-; in which any suitable H may be substituted by R⁶ and/or R⁷, and in which X is selected from NR⁸, O or S, while Y is selected from N or CH;
- =CH-X-Y-C(O)-; in which any suitable H may be substituted by R⁶ and in which one of X and Y is NR⁸, while the other is selected from NR^{8a}, S or O;
- =CH-X-Y-C(O)-; in which one of X and Y is CH₂, while the other is selected from NR⁸, S or O, in which any suitable H may be substituted by R⁶ and/or R⁷;
- =CR⁶-N=N-C(O)-;
- =CR⁹-CH=CH-CH=CH-; in which any suitable H may be substituted by R⁶;
- =CR⁹-CH=CH-CH=CR^{9a}-; in which any suitable H may be substituted by R⁶;
- =CH-X=Y-CH=CH-; in which any suitable H may be substituted by R⁶ and/or R⁷, and in which one of X or Y is selected from N, while the other is selected from N or CH;
- =CH-X=Y-CH₂-CH₂-; in which any suitable H may be substituted by R⁶ and/or R⁷, and in which one of X or Y is selected from N, while the other is selected from N or CH;
- =CH-X-Y-CH=CH-; in which any suitable H may be substituted by R⁶ and/or R⁷, and in which one of X or Y is selected from NR⁸, O or S while the other is selected from NR^{8a} or CH₂;
- =CH-X-Y-CH₂-CH₂-; in which any suitable H may be substituted by R⁶ and/or R⁷, and in which one of X or Y is selected from NR⁸, O or S while the other is selected from NR^{8a} or CH₂;
- =CH-X-CH₂-Y=CH-; in which any suitable H may be substituted by R⁶ and/or R⁷, and in which X is selected from NR⁸, O or S while Y is selected from N or CH;
- =CH-X-CH=Y-CH₂-; in which any suitable H may be substituted by R⁶ and/or R⁷, and in which X is selected from NR⁸, O or S while Y is selected from N or CH;
- =CH-N=CH-Y=CH-; in which any suitable H may be substituted by R⁶ and/or R⁷;
- =CH-X-CH₂-Y-CH₂-; in which any suitable H may be substituted by R⁶ and/or R⁷, and in which one of X or Y is selected from NR⁸, O or S while the other is selected from NR^{8a}, O, S or CH₂;

R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem;
Z is selected from
   - -(CH₂)ₙ-, with n being 1, 2, 3 or 4;
   - -O-(CH₂)ₙ-, with n being 1, 2, 3 or 4;
   - -S-(CH₂)ₙ-, with n being 1, 2, 3 or 4;
   - (CH₂)ₙ-(CHR⁵)-(CH₂)ₘ, with n and m being selected from 0, 1, 2 or 3 and m+n being 1, 2 or 3, with R⁵ being selected from F, Cl, Br, I, OH, SH, or unsubstituted C₁₋₄-Alkyl;
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the treatment of a 5-HT₇ mediated disease or condition.

In a preferred embodiment the use according to the invention relates to at least one compound according to formula Ia, 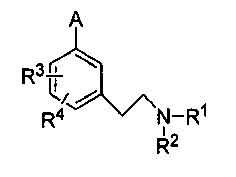 , wherein
A is a compound selected from the following group 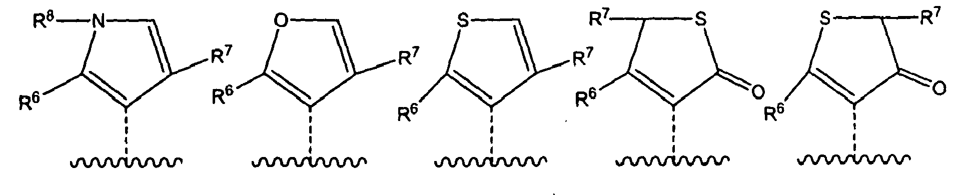 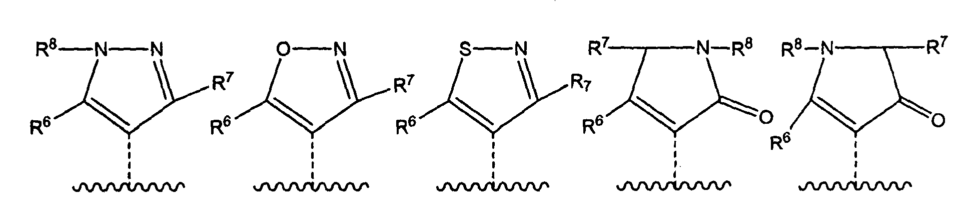 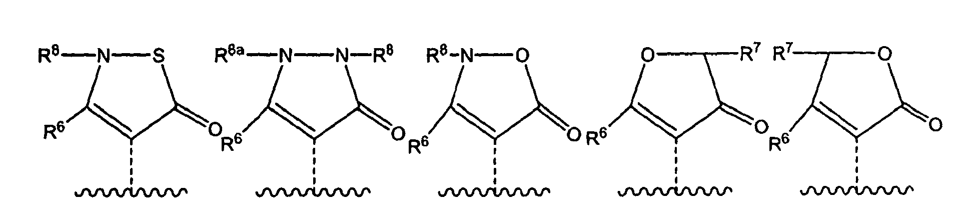 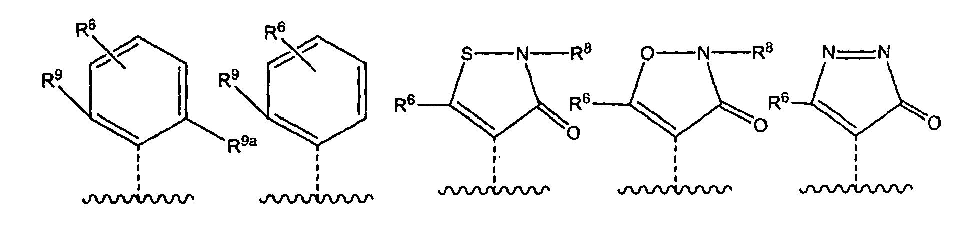
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the treatment of a 5-HT₇ mediated disease or condition.

In a preferred embodiment the use according to the invention relates to at least one compound according to formula la, 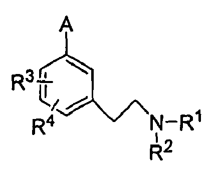 , wherein
A is a compound selected from the following group 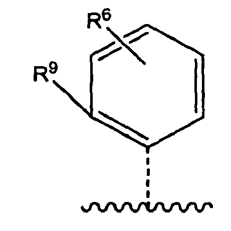 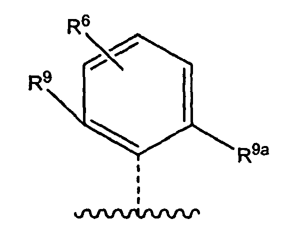
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ is selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the treatment of a 5-HT₇ mediated disease or condition.

The present invention also provides for the use of at least one compound according to the invention according to formula (I) or (Ia), optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the treatment of a 5-HT₇ mediated disease or condition.

In a preferred embodiment the use according to the invention relates to a use, wherein the disease is pain, preferably visceral pain, chronic pain, cancer pain, migraine, acute pain or neuropathic pain, more prefearably neuropathic pain, allodynia or hyperalgesia.

In another preferred embodiment the use according to the invention relates to a use, wherein the disease is sleep disorder, shift worker syndrome, jet lag, depression, seasonal affective disorder, migraine, anxiety, psychosis, schizophrenia, cognition and memory disorders, neuronal degeneration resulting from ischemic events, cardiovascular diseases such as hypertension, irritable bowel syndrome, inflammatory bowel disease, spastic colon or urinary incontinence.

The medicament/pharmaceutical composition may be in any form suitable for the application to humans and/or animals, preferably mammals, and can be produced by standard procedures known to those skilled in the art. The composition of the medicament may vary depending on the route of administration.

The medicament of the present invention may e.g. be administered parentally in combination with conventional injectable liquid carriers, such as water or suitable alcohols. Conventional pharmaceutical adjuvants for injection, such as stabilizing agents, solubilizing agents, and buffers, may be included in such injectable compositions. These medicaments may preferably be injected intramuscularly, intraperitoneally, or intravenously.

Medicaments according to the present invention may also be formulated into orally administrable compositions containing one or more physiologically compatible carriers or excipients, in solid or liquid form. These compositions may contain conventional ingredients such as binding agents, fillers, lubricants, and acceptable wetting agents. The compositions may take any convenient form, such as tablets, pellets, capsules, lozenges, aqueous or oily solutions, suspensions, emulsions, or dry powdered form suitable for reconstitution with water or other suitable liquid medium before use, for immediate or controlled release.

The liquid oral forms for administration may also contain certain additives such as sweeteners, flavoring, preservatives, and emulsifying agents. Non-aqueous liquid compositions for oral administration may also be formulated, containing e.g. edible oils. Such liquid compositions may be conveniently encapsulated in e.g., gelatin capsules in a unit dosage amount.

The compositions of the present invention may also be administered topically or via a suppository.

The above mentioned compositions include preferably 1 to 60 % by weight of one or more of the compound of general formula (I), optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and 40 to 99 % by weight of the appropriate pharmaceutical vehicle(s).

The daily dosage for humans and animals may vary depending on factors that have their basis in the respective species or other factors, such as age, weight or degree of illness and so forth. The daily dosage for mammals including humans usally ranges from 1 milligram to 2000 milligram, preferably 1 to 1500 mg, more preferably 1 to 1000 mg of substance to be administered during one or several intakes.

Thus, the invention also provides a method of treatment using the medicament/pharmaceutical compositions described above.

### Pharmacological Methods:

### Radioligand binding

Radioligand binding assays were performed using the Cloned Human Serotonin Receptor, Subtype 7 (h5HT₇), expressed in CHO cells, coated on Flashplate (Basic FlashPlate Cat.: SMP200) from PerkinElmer (Cat.: 6120512). The protocol assay was essentially the recommended protocol in the Technical Data Sheet by PerkinEmer Life and Analytical Sciences. The Mass membrane protein/well was typically 12 µg and the Receptor/well was about 9-10 fmoles. The Flashplate were let equilibrate at room temperature for one hour before the addition of the components of the assay mixture. The binding buffer was: 50 mM Tris-HCl, pH 7.4, containing 10 mM MgCl₂, 0.5 mM EDTA and 0.5% BSA. The radioligand was [¹²⁵I]LSD at a final concentration of 0.82 nM. Nonspecific binding was determined with 50 µM of Clozapine. The assay volume was 25 µl. TopSeal-A were applied onto Flashplate microplates and they were incubated at room temperature for 240 minutes in darkness. The radioactivity were quantified by liquid scintillation spectrophotometry (Wallac 1450 Microbeta Trilux) with a count delay of 4 minutes prior to counting and a counting time of 30 seconds per well. Competition binding data were analyzed by using the LIGAND program (Munson and Rodbard, LIGAND: A versatile, computerized approach for characterization of ligandbinding systems. Anal. Biochem. 107: 220-239, 1980) and assays were performed in triplicate determinations for each point.

Functionality assay on the 5HT7 receptor were done according to those known in the state of the art.

The following examples are given to illustrate the present invention, but they do not limit the scope of the present invention.

### EXAMPLES

A general scheme which was followed in general terms allowing for variants in preparing the examples is shown below:

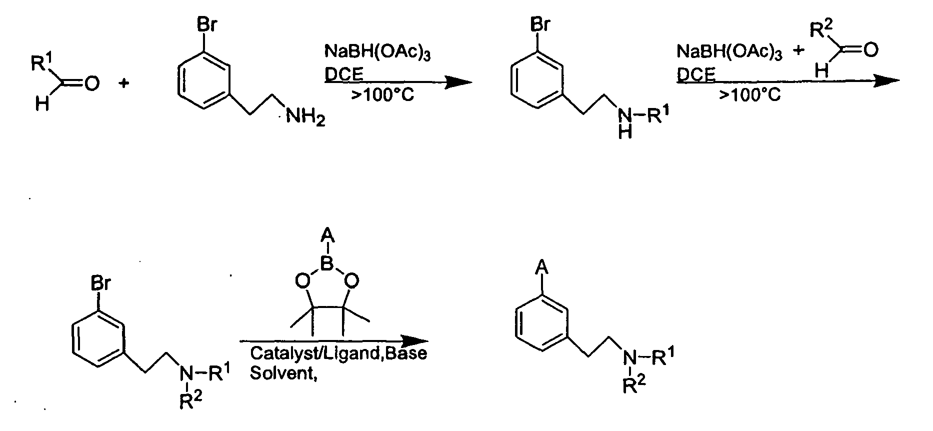

Base, Catalyst/ligand, solvent as well as temperature and reaction time could vary.

### Examples:

Prepared according to above-described methods.

### Example 1

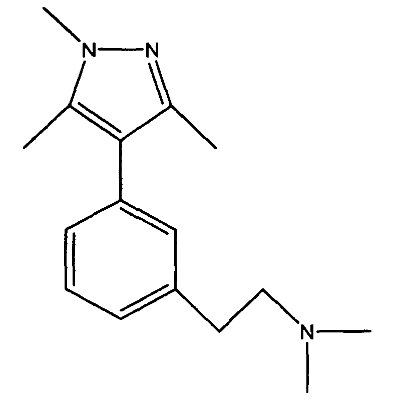

### Dimethyl-{2-[3-(1,3,5-trimethyl-1H-pyrazol-4-yl)-phenyl]-ethyl}-amine

In a more general form Example 1 was prepared according to the following Scheme

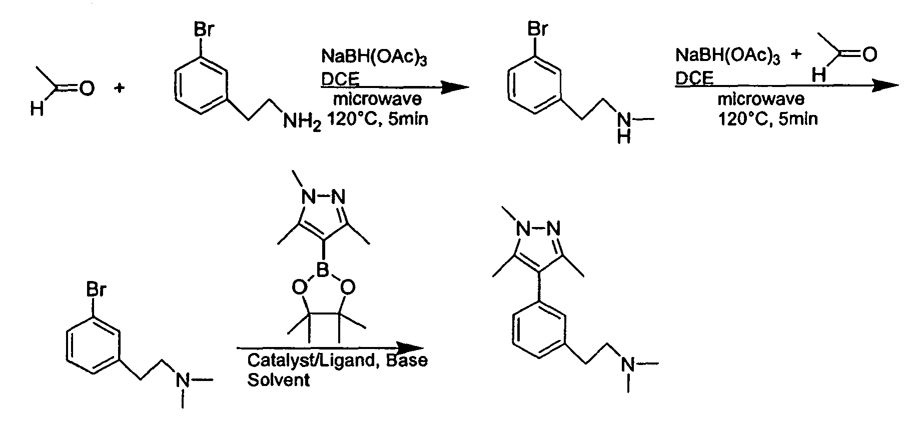

Base, Catalyst, Solvent, Temperature and reaction time in the last step, a so-called "Suzuki Reaction" were varied and in part orientated on literature such as JACS, 2002, 1162 and Angew. Chem. Int. Ed. 2006, 1282.

Thus the base was selected from K₂CO₃, K₃PO₄ and used in amounts between 1.7 and 5 eq. based on the amount of [2-(3-Bromo-phenyl)-ethyl]-dimethyl-amine introduced.
The Solvent was selected from DME/H₂O 1/1, and Dioxane/H₂O 2/1.
The Catalyst/ligand was selected from 1. (Pd₂(dba)₃, 4.3mol% + DPEPhos, 10mol%), 2. (Pd(PPh₃)₄, 10 mol%), 3. (Pd₂(dba)₃, 5 mol% + DPEPhos, 6 mol%) and 4.(Pd₂(dba)₃, 2 mol% + PCy₃' 4.8 mol%).
The Temperature was usually 10°C and the Reaction Time varied between a few minutes and 20 hours and even microwave irridation was used.
Yields varied between 8% and 78%.

After its precursors (A and B) were prepared Example 1 was synthesized following different methods explained more in detail below:

### Example A

### [2-(3-Bromo-phenyl)-ethyl]-methyl-amine

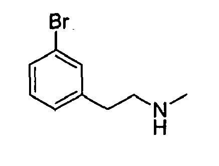

2-(3-Bromo-phenyl)-ethylamine (0.5 mmol) and formaldehyde (0.42 mmol) were mixed in 3 ml of 1,2-dichloroethane in a process vial, which was sealed with a septum. Sodium triacetoxyborohydride (0.84 mmol) was added under argon atmosphere. The suspension was subjected to microwave irradiating conditions (CEM Discover^{®} equipped with a CEM Explorer^{®} automated reaction handling module). The reaction mixture was heated for 5 min at 90°C and then cooled. The crude was evaporated to dryness and then suspended in aqueous NaHCO₃. The product was extracted with CH₂Cl₂ and washed with aqueous NaHCO₃. The CH₂Cl₂ extract was dried with anhydrous Na₂SO₄, filtered and evaporated to dryness to give the crude product [2-(3-bromo-phenyl)-ethyl]-methyl-amine. The crude was purified by flash column chromatography (CH₂Cl₂-MeOH as eluents) by using a CombiFlash Companion™ system to yield the title compound (75%) as colourless oil.

### Example B

### [2-(3-Bromo-phenyl)-ethyl]-dimethyl-amine

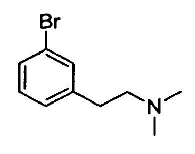

2-(3-Bromo-phenyl)-ethylamine (0.5 mmol) and formaldehyde (2.5 mmol) were mixed in 5 ml of 1,2-dichloroethane in a process vial, which was sealed with a septum. Sodium triacetoxyborohydride (1 mmol) was added under argon atmosphere. The suspension was subjected to microwave irradiating conditions (CEM Discover^{®} equipped with a CEM Explorer^{®} automated reaction handling module). The reaction mixture was heated for 5 min at 120°C and then cooled. The crude was evaporated to dryness and then suspended in aqueous NaHCO₃. The product was extracted with CH₂Cl₂ and washed with aqueous NaHCO₃. The CH₂Cl₂ extract was dried with anhydrous Na₂SO₄, filtered and evaporated to dryness to give the crude product [2-(3-bromo-phenyl)-ethyl]-dimethyl-amine. The crude was purified by flash column chromatography (CH₂Cl₂-MeOH as eluents) by using a CombiFlash Companion™ system to yield the title compound (86%) as colourless oil.

### Example 1

### Dimethyl-{2-[3-(1,3,5-trimethyl-1H-pyrazol-4-yl)-phenyl]-ethyl}-amine

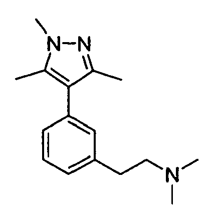

**(Method 1):** [2-(3-Bromo-phenyl)-ethyl]-dimethyl-amine (0.44 mmol) was dissolved in DME/H₂O 1/1 (8 mL) under argon atmosphere. 1,3,5-Trimethyl-1H-pyrazole-4-boronic acid pinacol ester (0.66 mmol), K₂CO₃ (2.19 mmol), and tetrakis-(triphenylphosphine)palladium (10 mol%, 0.044 mmol) were added and the reaction mixture was stirred at 100°C for 20h. The reaction mixture was evaporated to dryness, then dissolved in CHCl₃ and filtered through Celite® to give the crude product dimethyl-{2-[3-(1,3,5-trimethyl-1H-pyrazol-4-yl)-phenyl]-ethyl)-amine. The crude was purified by flash column chromatography (CH₂Cl₂-MeOH as eluents) by using a CombiFlash Companion™ system to yield the title compound (60%) as colourless oil.

**(Method 2):** [2-(3-Bromo-phenyl)-ethyl]-dimethyl-amine (0.22 mmol) was dissolved in DME/H₂O 1/1 (4 mL) under argon atmosphere in a process vial. 1,3,5-Trimethyl-1 H-pyrazole-4-boronic acid pinacol ester (0.33 mmol), K₂CO₃ (1.1 mmol), and tetrakis-(triphenylphosphine)palladium (10 mol%, 0.022 mmol) were added and the vial was sealed with a septum. The reaction mixture was subjected to microwave irradiating conditions, heated for 5 min at 100°C and then cooled. The reaction mixture was evaporated to dryness, then dissolved in CHCl₃ and filtered through Celite® to give the crude product dimethyl-{2-[3-(1,3,5-trimethyl-1H-pyrazol-4-yl)-phenyl]-ethyl}-amine. The crude was purified by flash column chromatography (CH₂Cl₂-MeOH as eluents) by using a CombiFlash Companion™ system to yield the title compound (78%) as colourless oil.

**(Method 3):** [2-(3-Bromo-phenyl)-ethyl]-dimethyl-amine (0.44 mmol), 1,3,5-trimethyl-1H-pyrazole-4-boronic acid pinacol ester (0.876 mmol), tris(dibenzylideneacetone)dipalladium (5 mol%, 0.022 mmol) and DPEPhos (6 mol%, 0.026 mmol) were dissolved in dioxane. K₃PO₄ (1.32 mmol) dissolved in 4 mL of water was added to the mixture and the reaction was stirred at 100°C for 20h. The reaction mixture was evaporated to dryness, then dissolved in CHCl₃ and filtered through Celite® to give the crude product dimethyl-{2-[3-(1,3,5-trimethyl-1H-pyrazol-4-yl)-phenyl]-ethyl}-amine. The crude was purified by flash column chromatography (CH₂Cl₂-MeOH as eluents) by using a CombiFlash Companion™ system to yield the title compound (24%) as colourless oil.

**(Method 4):** [2-(3-Bromo-phenyl)-ethyl]-dimethyl-amine (0.44 mmol), 1,3,5-trimethyl-1H-pyrazole-4-boronic acid pinacol ester (0526 mmol), tris(dibenzylideneacetone)dipalladium (2 mol%, 0.009 mmol) and tricyclohexylphosphine (4.8 mol%, 0.021 mmol) were dissolved in 4 mL of dioxane. K₃PO₄ (0.745 mmol) dissolved in 4 mL of water was added to the mixture and the reaction was stirred at 100°C for 20h. The reaction mixture was evaporated to dryness, then dissolved in CHCl₃ and filtered through Celite® to give the crude product dimethyl-{2-[3-(1,3,5-trimethyl-1H-pyrazol-4-yl)-phenyl]-ethyl}-amine. The crude was purified by flash column chromatography (CH₂Cl₂-MeOH as eluents) by using a CombiFlash Companion™ system to yield the title compound (8%) as colourless oil.

**(Method 5):** [2-(3-Bromo-phenyl)-ethyl]-dimethyl-amine (0.44 mmol), 1,3,5-trimethyl-1H-pyrazole-4-boronic acid pinacol ester (0526 mmol), tris(dibenzylideneacetone)dipalladium (4.3 mol%, 0.019 mmol) and DPEPhos (10 mol%, 0.044 mmol) were dissolved in 4 mL of dioxane. K₃PO₄ (2.19 mmol) dissolved in 4 mL of water was added to the mixture and the reaction was stirred at 100°C for 20h. The reaction mixture was evaporated to dryness, then dissolved in CHCl₃ and filtered through Celite® to give the crude product dimethyl-{2-[3-(1,3,5-trimethyl-1H-pyrazol-4-yl)-phenyl]-ethyl}-amine. The crude was purified by flash column chromatography (CH₂Cl₂-MeOH as eluents) by using a CombiFlash Companion™ system to yield the title compound (34%) as colourless oil.

The other examples were or are prepared according to or analogous to the reaction schemes and descriptions given above and are listed (where applicable) together with their binding data, their Mass and their 1 H-NMR data in the following table:

| **Example** | **Structure** | **Name** | **IC50 (nM)** | **% inh (10⁻** | **% inh (10^{- 7}M)** | **% inh (10^{- 8}M)** | **¹H-NMR** | **MS (APCI (M+H)⁺)** |
|---|---|---|---|---|---|---|---|---|
| **1** | 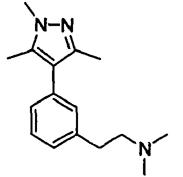 | Dimethyl-{2-[3-(1,3,5-trimethyl-1H-pyrazol-4-yl)-phenyl]-ethyl}-amine | 6.1 | | 98.6 | 60.8 | (300 MHz, METHANOL-*d*₄) δ ppm 2.18 (d, *J*=2.78 Hz, 3 H) 2.25 (d, *J*=2.64 Hz, 3 H) 2.37 (d, *J*=2.64 Hz, 6 H) 2.61 - 2.70 (m, 2 H) 2.81 - 2.90 (m, 2 H) 3.76 (d, *J*=2.64 Hz, 3 H) 7.07 - 7.13 (m, 2 H) 7.17 (d, *J*=7.47 Hz, 1 H) 731-739 (m 1H) | 258.08 |
| **2** | 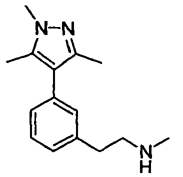 | Methyl-{2-[3-(1,3,5-trimethyl-1H-pyrazol-4-yl)-phenyl]-ethyl}-amine | | | | | | |
| **3** | 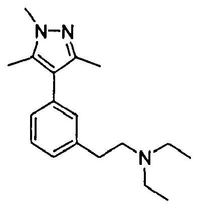 | Diethyl-{2-[3-(1,3,5-trimethyl-1H-pyrazol-4- yl)-phenyl]-ethyl}-amine | | | | | | 286.18 |
| **4** | 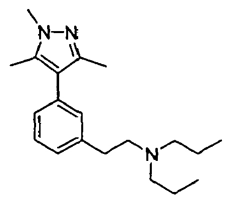 | Dipropyl-{2-[3-(1,3,5-trimethyl-1 H-pyrazol-4-yl)-phenyl]-ethyl}-amine | | | | | | 314.2 |
| **5** | 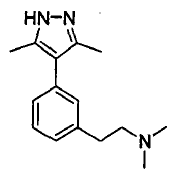 | {2-[3-(3,5-Dimethyl-1H-pyrazol-4-yl)-phenyl]-ethyl}-dimethyl-amine | | | | | | |
| **6** | 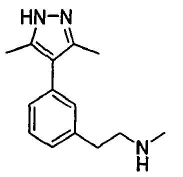 | {2-[3-(3,5-Dimethyl-1H-pyrazol-4-yl)-phenyl]-ethyl}-methyl-amine | | | | | | |
| **7** | 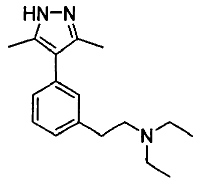 | {2-[3-(3,5-Dimethyl-1H-pyrazol-4-yl)-phenyl]-ethyl}-diethyl-amine | | | | | | |
| **8** | 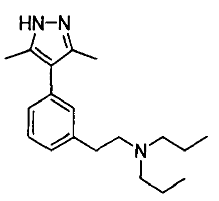 | {2-[3-(3,5-Dimethyl-1H-pyrazol-4-yl)-phenyl]-ethyl}-dipropyl-amine | | | | | | |
| **9** | 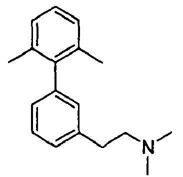 | [2-(2',6'-Dimethylbiphenyl-3-yl)-ethyl]-dimethyl-amine | | 104.6 | 88.9 | 65.9 | (400 MHz, METHANOL-*d*₄) δ ppm 1.98 (s, 6 H) 2.33 (s, 6 H) 2.58-2.65 (m, 2 H) 2.81-2.87 (m, 2 H) 6.93- 6.99(m, 2 H) 7.04 - 7.13 (m, 3 H) 7.21 (d, *J*=7.82 Hz, 1 H) 7.36 (t, J=7.62 Hz, 1 H) | 254.25 |
| **10** | 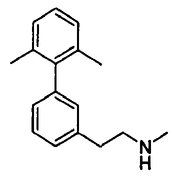 | [2-(2',6'-Dimethylbiphenyl-3-yl)-ethyl]-methyl-amine | | | | | | |
| **11** | 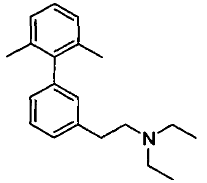 | [2-(2',6'-Dimethylbiphenyl-3-yl)-ethyl]-diethyl-amine | | | | | | 282.1 |
| **12** | 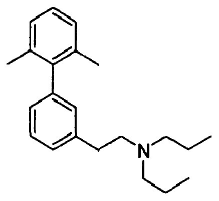 | [2-(2',6'-Dimethylbiphenyl-3-yl)-ethyl]- dipropyl-amine | | | | | | 310.28 |
| **13** | 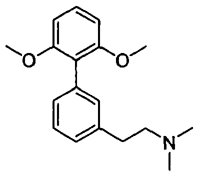 | [2-(2',6'-Dimethoxybiphenyl-3-yl)-ethyl]-dimethyl-amine | | | | | | 286.14 |
| **14** | 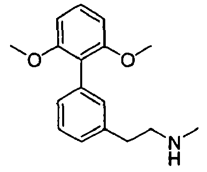 | [2-(2',6'-Dimethoxybiphenyl-3-yl)-ethyl]-methyl-amine | | | | | | |
| **15** | 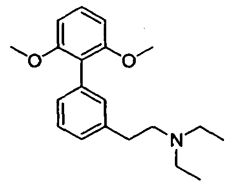 | [2-(2',6'-Dimethoxybiphenyl-3-yl)-ethyl]-diethyl-amine | | | | | | 314.15 |
| **16** | 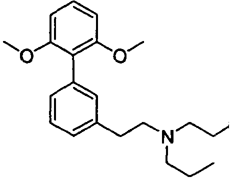 | [2-(2',6'-Dimethoxybiphenyl-3-yl)-ethyl]-dipropyl-amine | | | | | | 342.27 |
| **17** | 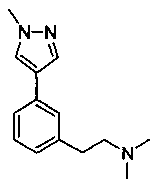 | Dimethyl-{2-[3-(1-methyl-1 H-pyrazol-4-yl)-phenyl]-ethyl}-amine | | 46 | 16.9 | -1 | | 230.32 |
| **18** | 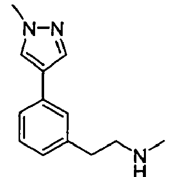 | Methyl-{2-[3-(1-methyl-1H-pyrazol-4-yl)-phenyl]-ethyl}-amine | | | | | | |
| **19** | 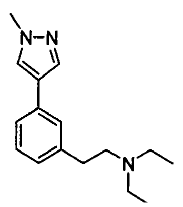 | Diethyl-{2-[3-(1-methyl-1 H-pyrazol-4-yl)-phenyl]-ethyl}-amine | | | | | | 258.20 |
| **20** | 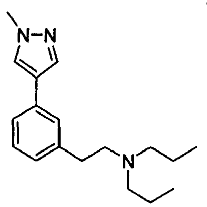 | {2-[3-(1-Methyl-1H-pyrazol-4-yl)-phenyl]-ethyl}-dipropyl-amine | | | | | | 286.23 |
| **21** | 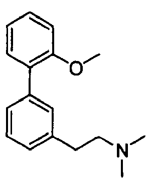 | [2-(2'-Methoxybiphenyl-3-yl)ethyl]-dimethyl-amine | | 95.4 | 69.2 | 20 | (400 MHz, METHANOL-*d*₄) δ ppm 2.33 (s, 6 H) 2.59 - 2.65 (m, 2 H) 2.80-2.86 (m, 2 H) 3.77 (s, 3 H) 6.99 (td, *J*=7.42, 1.17 Hz, 1H) 7.04 (d, *J*=7.82 Hz, 1 H) 7.15 (m, 1 H) 7.23 - 7.32 (m, 5 H) | 256.25 |
| **22** | 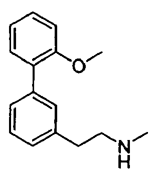 | [2-(2'-Methoxybiphenyl-3-yl)-ethyl]-methyl-amine | | | | | | |
| **23** | 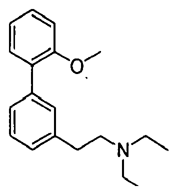 | Diethyl-[2-(2'-methoxybiphenyl-3-yl)-ethyl]-amine | | | | | | 284.21 |
| **24** | 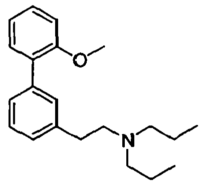 | [2-(2'-Methoxybiphenyl-3-yl)-ethyl]-dipropyl-amine | | | | | | 312.22 |
| **25** | 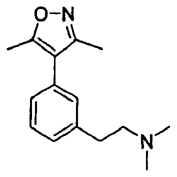 | {2-[3-(3,5-Dimethylisoxazol-4-yl)-phenyl]-ethyl}-dimethyl-amine | | | | | | 245.20 |
| **26** | 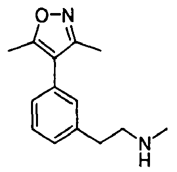 | {2-[3-(3,5-Dimethylisoxazo)-4-yl)-phenyl]-ethyl}-methyl-amine | | | | | | |
| **27** | 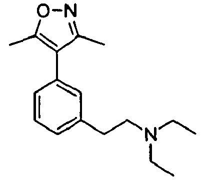 | {2-[3-(3,5-Dimethylisoxazol-4-yl)-phenyl]-ethyl}-diethyl-amine | | | | | | 273.16 |
| **28** | 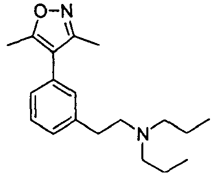 | {2-[3-(3,5-Dimethylisoxazol-4-yl)-phenyl]-ethyl}-dipropyl-amine | | | | | | 301.31 |

### Pharmacological Data:

Results for representative compounds/examples are given in the table below:

| **COMPOUND EXAMPLE** | **5-HT₇ IC50 (nM)** | **5-HT₇ EC50 (nM)** | **5-HT₇ Emax (%)** | **5-HT₆ inhib.(10-⁷M)** (%) | **5-HT₁ IC50 (nM)** |
|---|---|---|---|---|---|
| 1 | 6.1 ±0.7 | 25.5±2 | 94 % | 5.4% | > 1000 |
| | | | | | |
| | | | | | |
| | | | | | |
| | | | | | |

### Formulation Example

### Example of a tablet formulation:

| | |
|---|---|
| Compound according to example 1 | 5 mg |
| Lactose | 60 mg |
| Crystalline cellulose | 25 mg |
| Povidone K 90 | 5 mg |
| Pregelanitized starch | 3 mg |
| Colloidal silica dioxide | 1 mg |
| Magnesium stearate | 1 mg |
| Total weight per tablet | 100 mg |

The above mentioned ingredients were mixed and compressed into a tablet by conventional methods known to those skilled in the art.

## Claims

1. Heterocyclyl-substituted-ethylamino-phenyl derivative of general formula (I) 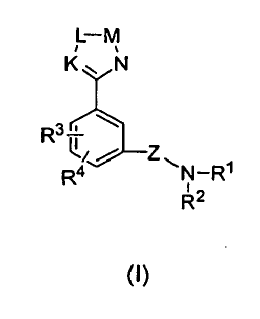 wherein
K-L-M-N together form
• =CH-X-Y=CH-; in which any suitable H may be substituted by R⁶ and/or R⁷, and in which X is selected from NR⁸, O or S, while Y is selected from N or CH;
• =CH-X-Y-C(O)-; in which any suitable H may be substituted by R⁶ and in which one of X and Y is NR⁸, while the other is selected from NR^{8a}, S or O;
• =CH-X-Y-C(O)-; in which one of X and Y is CH₂, while the other is selected from NR⁸, S or O, in which any suitable H may be substituted by R⁶ and/or R⁷;
• =CR⁶-N=N-C(O)-;
• =CR⁹-CH=CH-CH=CH-; in which any suitable H may be substituted by R⁶;
• =CR⁹-CH=CH-CH=CR^{9a}-; in which any suitable H may be substituted by R⁶;
• =CH-X=Y-CH=CH-; in which any suitable H may be substituted by R⁶ and/or R⁷, and in which one of X or Y is selected from N, while the other is selected from N or CH;
• =CH-X=Y-CH₂-CH₂-; in which any suitable H may be substituted by R⁶ and/or R⁷, and in which one of X or Y is selected from N, while the other is selected from N or CH;
• =CH-X-Y-CH=CH-; in which any suitable H may be substituted by R⁶ and/or R⁷, and in which one of X or Y is selected from NR⁸, O or S while the other is selected from NR^{8a} or CH₂;
• =CH-X-Y-CH₂-CH₂-; in which any suitable H may be substituted by R⁶ and/or R⁷, and in which one of X or Y is selected from NR⁸, O or S while the other is selected from NR^{8a} or CH₂;
• =CH-X-CH₂-Y=CH-; in which any suitable H may be substituted by R⁶ and/or R⁷, and in which X is selected from NR⁸, O or S while Y is selected from N or CH;
• =CH-X-CH=Y-CH₂-; in which any suitable H may be substituted by R⁶ and/or R⁷, and in which X is selected from NR⁸, O or S while Y is selected from N or CH;
• =CH-N=CH-Y=CH-; in which any suitable H may be substituted by R⁶ and/or R⁷;
• =CH-X-CH₂-Y-CH₂-; in which any suitable H may be substituted by R⁶ and/or R⁷, and in which one of X or Y is selected from NR⁸, O or S while the other is selected from NR^{8a}, O, S or CH₂;
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem;
Z is selected from
• -(CH₂)ₙ-, with n being 1, 2, 3 or 4;
• -O-(CH₂)ₙ-, with n being 1, 2, 3 or 4;
• -S-(CH₂)n-, with n being 1, 2, 3 or 4;
• (CH₂)ₙ-(CHR⁵)-(CH₂)ₘ, with n and m being selected from 0, 1, 2 or 3 and m+n being 1, 2 or 3, with R⁵ being selected from F, Cl, Br, I, OH, SH, or unsubstituted C₁₋₄-Alkyl;
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers, preferably enantiomers or diastereomers, in any mixing ratio, or a salt, preferably a physiologically acceptable salt thereof, or a corresponding solvate, respectively;
• with the proviso that
if R¹and R² are both CH₃, R³ and R⁴ are both H, K-L-M-N together form =CR⁹-CH=CH-CH=CR^{9a}- and one of R⁹ or R^{9a} is -CH=CH₂, the other may not be OCH₃;
• and with the proviso that
if R¹ and R² are both H, one of R³ and R⁴ is H, while the other is -O(C₂H₅), K-L-M-N together form =CR⁹-CR⁶=CH-CH=CH-, and R⁹ is -OCH₃, R⁶ may not be OCH₃.

2. Compound according to claim 1, **characterized in that** the compound is a compound according to Formula la 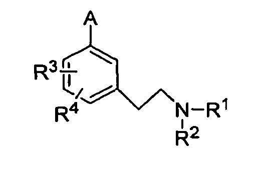 , wherein
A is a compound selected from the following group 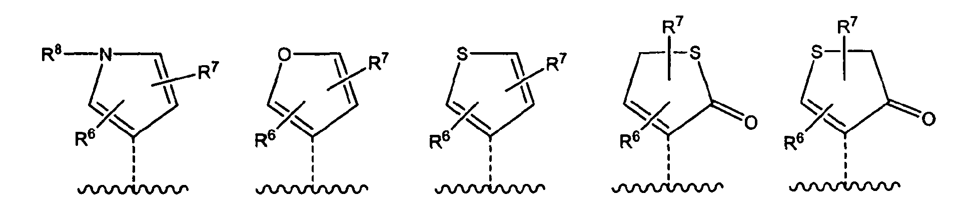 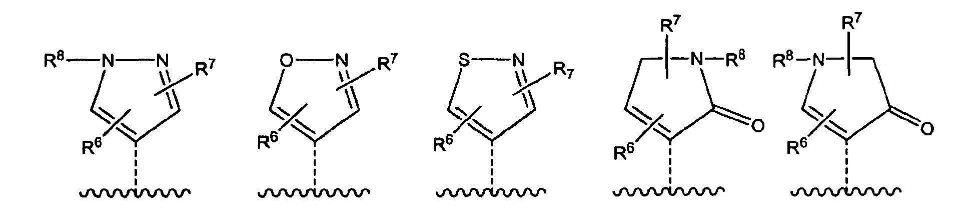 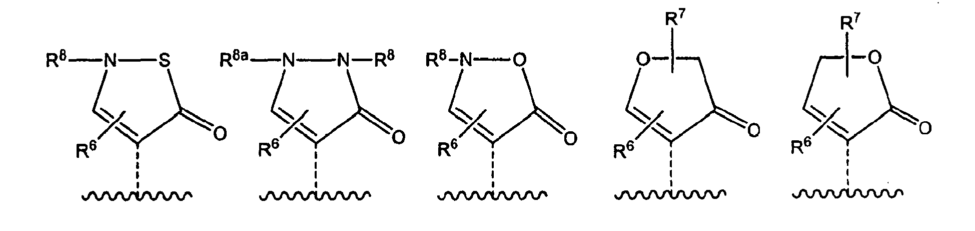 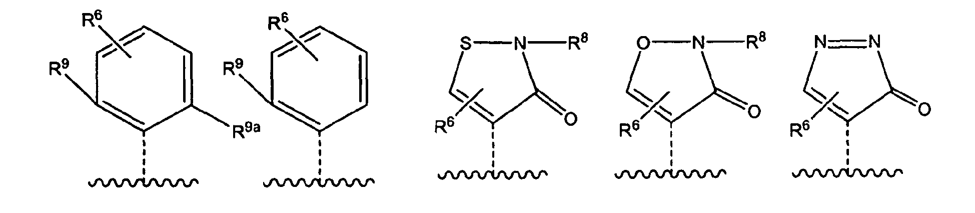
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
• with the proviso that
if R'and R² are both CH₃, R³ and R⁴ are both H, A is 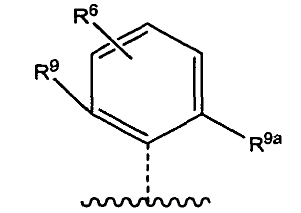 and one of R⁹ or R^{9a} is -CH=CH₂, the other may not be OCH₃;
• and with the proviso that
if R¹ and R² are both H, one of R³ and R⁴ is H, while the other is -O(C₂H₅), A is 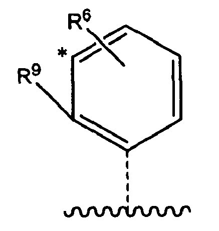 and R⁹ is -OCH₃, R⁶ may not be OCH₃ in the position marked with "*".

3. Compound according to claim 2, **characterized in that** the compound is a compound according to Formula Ia, wherein
A is a compound selected from the following group 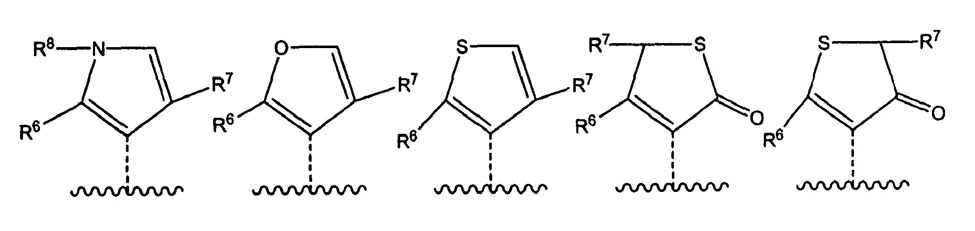 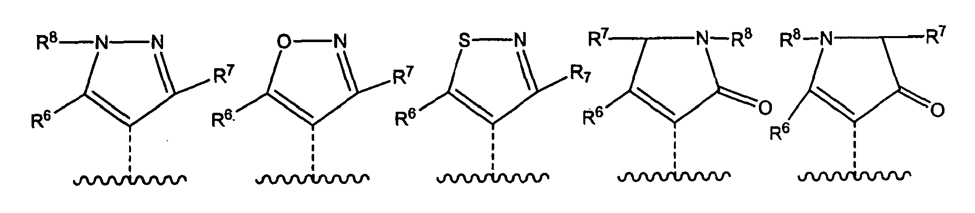 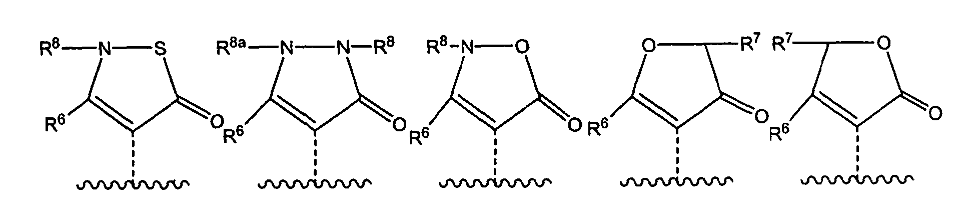 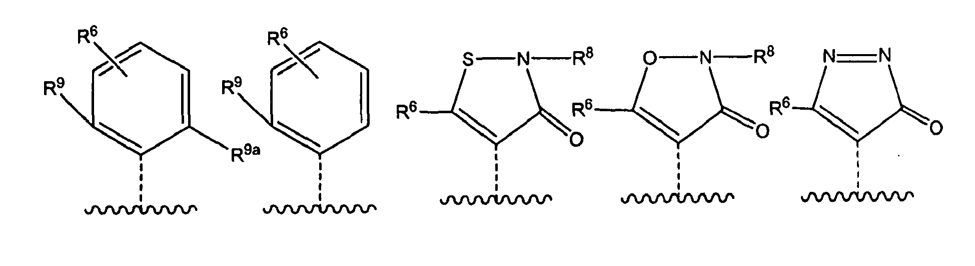
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
• with the proviso that
if R¹ and R² are both CH₃, R³ and R⁴ are both H, A is 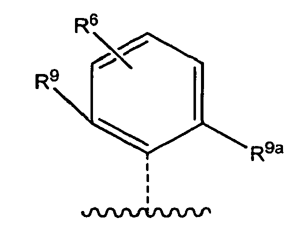 and one of R⁹ or R^{9a} is -CH=CH₂, the other may not be OCH₃;
• and with the proviso that
if R¹ and R² are both H, one of R³ and R⁴ is H, while the other is -O(C₂H₅), A is 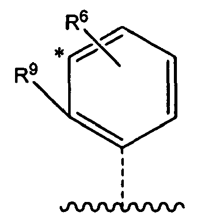 and R⁹ is -OCH₃, R⁶ may not be OCH₃ in the position marked with "*".

4. Compound according to claim 2 or 3, **characterized in that** the compound is a compound according to Formula Ia, wherein
A is a compound selected from the following group 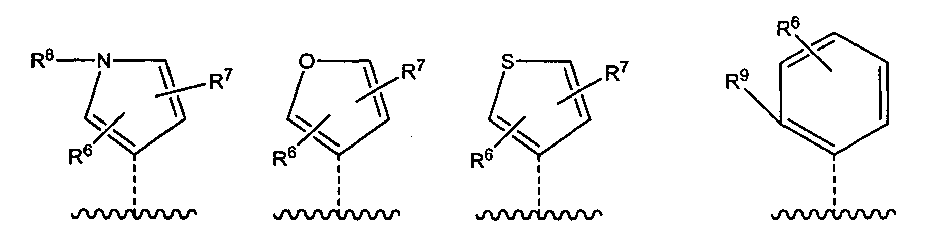 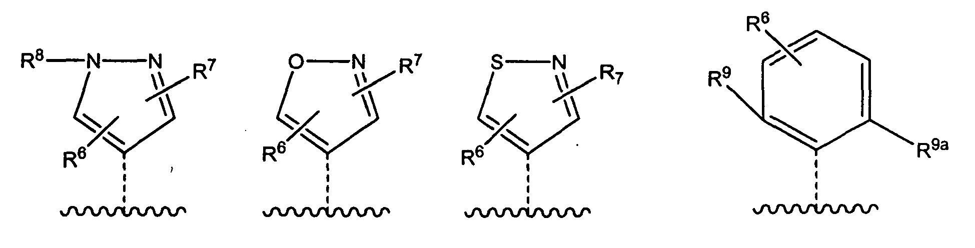 or 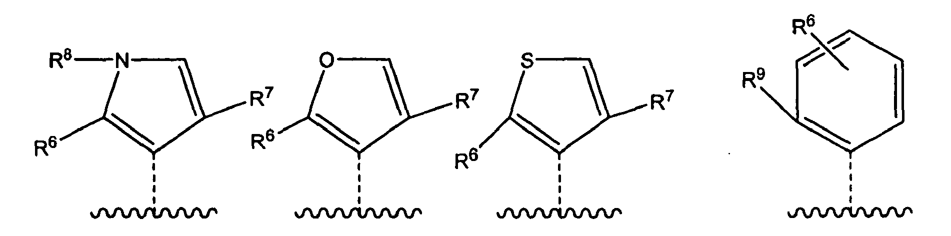 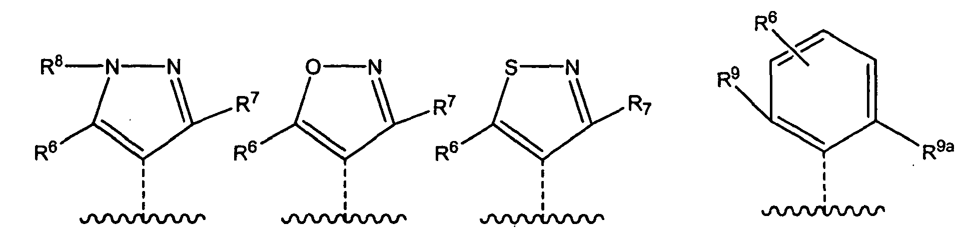
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
• with the proviso that
if R¹ and R² are both CH₃, R³ and R⁴ are both H, A is 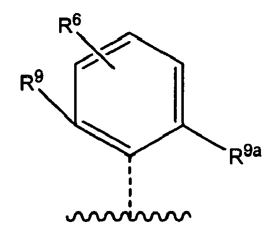 and one of R⁹ or R^{9a} is -CH=CH₂, the other may not be OCH₃;
• and with the proviso that
if R¹ and R² are both H, one of R³ and R⁴ is H, while the other is -O(C₂H₅), A is 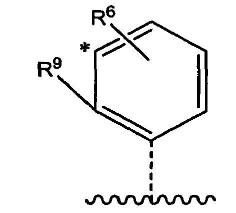 and R⁹ is -OCH₃, R⁶ may not be OCH₃ in the position marked with "*".

5. Compound according to claim 4, **characterized in that** the compound is a compound according to Formula la, wherein
A is a compound selected from the following group 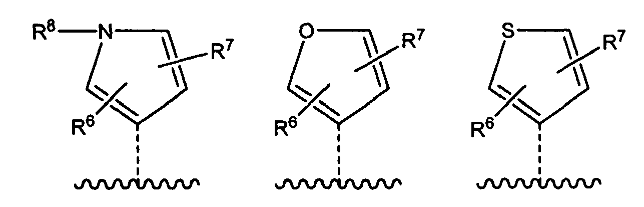 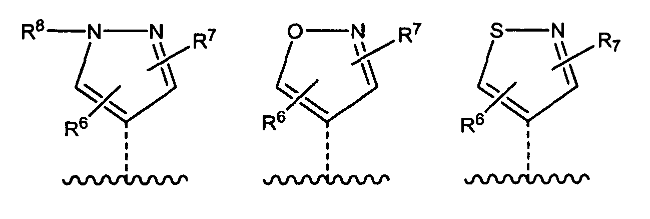 or 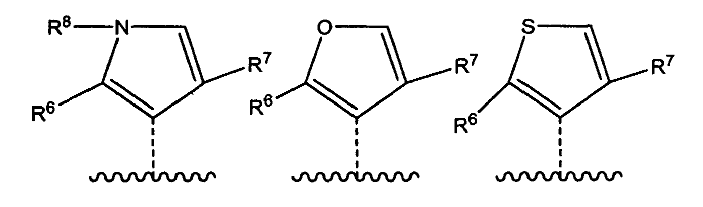 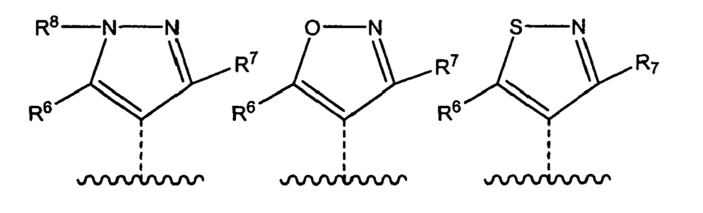
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem;
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ is selected from hydrogen; or an aliphatic radical, which is linear or branched, saturated or unsaturated, and optionally at least mono-substituted by F, Cl, Br, I, SH or OH.

6. Compound according to claim 4, **characterized in that** the compound is a compound according to Formula la, wherein
A is a compound selected from the following group 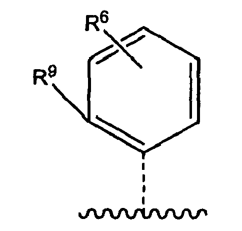 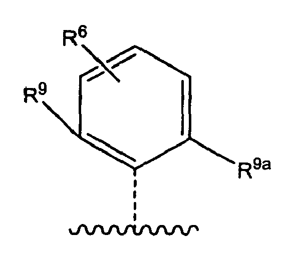
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ is selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
• with the proviso that
if R¹ and R² are both CH₃, R³ and R⁴ are both H, A is 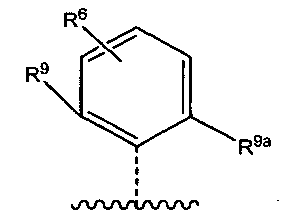 and one of R⁹ or R^{9a} is -CH=CH₂, the other may not be OCH₃;
• and with the proviso that
if R¹ and R² are both H, one of R³ and R⁴ is H, while the other is -O(C₂H₅), A is 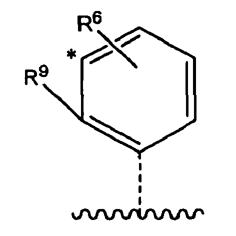
and R⁹ is -OCH₃, R⁶ may not be OCH₃ in the position marked with "*".

7. Compound according to any one of claims 1 to 6, **characterized in that**
R¹ and R² each are independently selected from the group consisting of hydrogen; or a linear or branched, optionally at least mono-substituted C₁₋₄-alkyl radical; or
R¹ and R² together with the bridging nitrogen atom form an saturated or unsaturated, optionally at least mono-substituted 5- or 6-membered-heterocyclic ring;
preferably **in that**
R¹ and R² are each independently selected from the group consisting of hydrogen; or a linear or branched C₁₋₄-alkyl radical; or
R¹ and R² together with the bridging nitrogen atom form an saturated or unsaturated, optionally at least mono-substituted 5- or 6-membered-heterocyclic ring;
more preferably **in that**
R¹ and R² are each independently selected from the group consisting of hydrogen, CH₃, C₂H₅ or C₃H₇.

8. Compound according to any one of claims 1 to 7, **characterized in that**
R³ and R⁴ are independently from each other selected from hydrogen; halogen, OH, SH, NH₂; a linear or branched, optionally at least mono-substituted C₁₋₄-alkyl radical; or O-R with R being a linear or branched, optionally at least mono-substituted C₁₋₄-alkyl radical;
preferably **in that**
R³ and R⁴ are independently from each other selected from H, F, Cl, Br, I, OH, SH, NH₂, CH₃, C₂H₅, C₃H₇, C₄H₉, OCH₃, OC₂H₅, OC₃H₇ or OC₄H₉,
more preferably **in that**
R³ and R⁴ are H.

9. Compound according to any one of claims 1 to 5, **characterized in that**
R⁶ and R⁷ are independently from each other selected from hydrogen; halogen, OH, SH, NH₂; a C₁₋₄-alkyl radical, which is linear or branched, and optionally at least mono-substituted by F, Cl, Br, I, SH or OH; or O-R with R being a C₁₋₄-alkyl radical, which is linear or branched, and optionally at least mono-substituted by F, Cl, Br, I, SH or OH;
preferably **in that**
R⁶ and R⁷ are independently from each other selected from H, F, Cl, Br, I, OH, SH, NH₂, CH₃, C₂H₅, C₃H₇, C₄H₉, OCH₃, OC₂H₅, OC₃H₇ or OC₄H₉;
more preferably **in that**
R⁶ and R⁷ are independently from each other selected from H, or CH₃.

10. Compound according to any one of claims 1 to 5, **characterized in that**
R⁸ is selected from hydrogen; or a C₁₋₄-alkyl radical, which is linear or branched, and optionally at least mono-substituted by F, Cl, Br, I, SH or OH;
preferably **in that**
R⁸ is selected from H, F, Cl, Br, I, OH, SH, NH₂, CH₃, C₂H₅, C₃H₇, or C₄H₉;
more preferably **in that**
R⁸ is selected from H or CH₃.

11. Compound according to any one of claims 1 to 5, selected from
• Dimethyl-{2-[3-(1,3,5-trimethyl-1H-pyrazol-4-yl)-phenyl]-ethyl}-amine,
• Methyl-{2-[3-(1,3,5-trimethyl-1H-pyrazol-4-yl)-phenyl]-ethyl}-amine,
• Diethyl-{2-[3-(1,3,5-trimethy)-1H-pyrazol-4-yl)-phenyl]-ethyl}-amine,
• Dipropyl-{2-[3-(1,3,5-trimethyl-1H-pyrazol-4-yl)-phenyl]-ethyl}-amine,
• {2-[3-(3,5-Dimethyl-1H-pyrazol-4-yl)-phenyl]-ethyl}-dimethyl-amine,
• {2-[3-(3,5-Dimethyl-1H-pyrazol-4-yl)-phenyl]-ethyl}-methyl-amine,
• {2-[3-(3,5-Dimethyl-1H-pyrazol-4-yl)-phenyl]-ethyl}-diethyl-amine,
• {2-[3-(3,5-Dimethyl-1H-pyrazol-4-yl)-phenyl]-ethyl}-dipropyl-amine,
• Dimethyl-{2-[3-(1-methyl-1H-pyrazol-4-yl)-phenyl]-ethyl}-amine,
• Methyl-{2-[3-(1-methyl-1H-pyrazol-4-yl)-phenyl]-ethyl}-amine,
• Diethyl-{2-[3-(1-methyl-1H-pyrazol-4-yl)-phenyl]-ethyl}-amine,
• {2-[3-(1-Methyl-1H-pyrazol-4-yl)-phenyl]-ethyl}-dipropyl-amine,
• {2-[3-(3,5-Dimethyl-isoxazol-4-yl)-phenyl]-ethyl}-dimethyl-amine,
• {2-[3-(3,5-Dimethyl-isoxazol-4-yl)-phenyl]-ethyl}-methyl-amine,
• {2-[3-(3,5-Dimethyl-isoxazol-4-yl)-phenyl]-ethyl}-diethyl-amine, or
• {2-[3-(3,5-Dimethyl-isoxazol-4-yl)-phenyl]-ethyl}-dipropyl-amine,
optionally in form of a salt, preferably a physiologically acceptable salt, more preferably in form of a physiologically acceptable acid addition salt, most preferably a hydrochloride salt, or a corresponding solvate.

12. Compound according to any one of claims 1 to 4 and 6, **characterized in that**
R⁶ is selected from hydrogen; halogen, OH, SH, NH₂; a C₁₋₄-alkyl radical, which is linear or branched, and optionally at least mono-substituted by F, Cl, Br, I, SH or OH; or O-R with R being a C₁₋₄-alkyl radical, which is linear or branched, and optionally at least mono-substituted by F, Cl, Br, I, SH or OH;
preferably **in that**
R⁶ is selected from H, F, Cl, Br, I, OH, SH, NH₂, CH₃, C₂H₅, C₃H₇, C₄H₉, OCH₃, OC₂H₅, OC₃H₇ or OC₄H₉;
more preferably **in that**
R⁶ is H.

13. Compound according to any one of claims 1 to 4 and 6, **characterized in that**
R⁹ and R^{9a} are independently from each other selected from a C₁₋₄-alkyl radical, which is linear or branched, and optionally at least mono-substituted by F, Cl, Br, I, SH or OH; or O-R with R being a C₁₋₄-alkyl radical, which is linear or branched, and optionally at least mono-substituted by F, Cl, Br, I, SH or OH;
preferably **in that**
R⁹ and R^{9a} are independently from each other selected from CH₃, C₂H₅, C₃H₇, C₄H₉, OCH₃, OC₂H₅, OC₃H₇ or OC₄H₉;
more preferably **in that**
R⁹ and R^{9a} are independently from each other selected from CH₃, or OCH₃.
most preferably **in that**
R⁹ and R^{9a} are both selected either from CH₃, or OCH₃.

14. Compounds according to one or more of claims 1 to 4 and 6, selected from
• [2-(2',6'-Dimethyl-biphenyl-3-yl)-ethyl]-dimethyl-amine,
• [2-(2',6'-Dimethyl-biphenyl-3-yl)-ethyl]-methyl-amine,
• [2-(2',6'-Dimethyl-biphenyl-3-yl)-ethyl]-diethyl-amine,
• [2-(2' ,6'-Dimethyl-biphenyl-3-yl)-ethyl]-dipropyl-amine,
• [2-(2',6'-Dimethoxy-biphenyl-3-yl)-ethyl]-dimethyl-amine,
• [2-(2',6'-Dimethoxy-biphenyl-3-yl)-ethyl]-methyl-amine,
• [2-(2',6'-Dimethoxy-biphenyl-3-yl)-ethyl]-diethyl-amine,
• [2-(2',6'-Dimethoxy-biphenyl-3-yl)-ethyl]-dipropyl-amine,
• [2-(2'-Methoxy-biphenyl-3-yl)-ethyl]-dimethyl-amine,
• [2-(2'-Methoxy-biphenyl-3-yl)-ethyl]-methyl-amine,
• Diethyl-[2-(2'-methoxy-biphenyl-3-yl)-ethyl]-amine, or
• [2-(2'-Methoxy-biphenyl-3-yl)-ethyl]-dipropyl-amine,
optionally in form of a salt, preferably a physiologically acceptable salt, more preferably in form of a physiologically acceptable acid addition salt, most preferably a hydrochloride salt, or a corresponding solvate.

15. Process for the preparation of compounds according to claim 1, **characterized in that** a compound of general formula (VI) 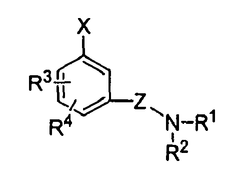 , wherein R¹, R², R³, R⁴ and Z are as defined in claim 1, and X represents halogen, preferably Br, or an O-triflate group, is reacted with a compound of general formula VII or VIIa 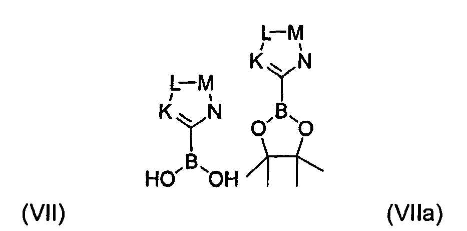 , wherein K, L, M, and N are as defined in claim 1, to form a compound according to formula I, preferably in presence of a catalyst.

16. Process according to claim 15, **characterized in that**
a) the catalyst is a palladium catalyst with or without a ligand, and/or
b) the reaction is carried out in presence of at least one base, selected from organic or inorganic bases and/or
c) the reaction is carried out in a suitable reaction medium selected from ethers, alcohols, hydrocarbons or other organic solvents.

17. Medicament comprising at least one compound according to formula 1, 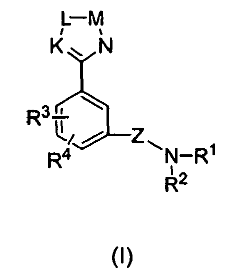 wherein
K-L-M-N together form
• =CH-X-Y=CH-; in which any suitable H may be substituted by R⁶ and/or R⁷, and in which X is selected from NR⁸, O or S, while Y is selected from N or CH;
• =CH-X-Y-C(O)-; in which any suitable H may be substituted by R⁶ and in which one of X and Y is NR⁸, while the other is selected from NR^{8a}, S or O;
• =CH-X-Y-C(O)-; in which one of X and Y is CH₂, while the other is selected from NR⁸, S or O, in which any suitable H may be substituted by R⁶ and/or R⁷;
• =CR⁶-N=N-C(O)-;
• =CR⁹-CH=CH-CH=CH-; in which any suitable H may be substituted by R⁶;
• =CR⁹-CH=CH-CH=CR^{9a}-; in which any suitable H may be substituted by R⁶;
• =CH-X=Y-CH=CH-; in which any suitable H may be substituted by R⁶ and/or R⁷, and in which one of X or Y is selected from N, while the other is selected from N or CH;
• =CH-X=Y-CH₂-CH₂-; in which any suitable H may be substituted by R⁶ and/or R⁷, and in which one of X or Y is selected from N, while the other is selected from N or CH;
• =CH-X-Y-CH=CH-; in which any suitable H may be substituted by R⁶ and/or R⁷, and in which one of X or Y is selected from NR⁸, O or S while the other is selected from NR^{8a} or CH₂;
• =CH-X-Y-CH₂-CH₂-; in which any suitable H may be substituted by R⁶ and/or R⁷, and in which one of X or Y is selected from NR⁸, O or S while the other is selected from NR^{8a} or CH₂;
• =CH-X-CH₂-Y=CH-; in which any suitable H may be substituted by R⁶ and/or R⁷, and in which X is selected from NR⁸, O or S while Y is selected from N or CH;
• =CH-X-CH=Y-CH₂-; in which any suitable H may be substituted by R⁶ and/or R⁷, and in which X is selected from NR⁸, O or S while Y is selected from N or CH;
• =CH-N=CH-Y=CH-; in which any suitable H may be substituted by R⁶ and/or R⁷;
• =CH-X-CH₂-Y-CH₂-; in which any suitable H may be substituted by R⁶ and/or R⁷, and in which one of X or Y is selected from NR⁸, O or S while the other is selected from NR^{8a}, O, S or CH₂;
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem;
Z is selected from
• -(CH₂)ₙ-, with n being 1, 2, 3 or 4;
• -O-(CH₂)ₙ-, with n being 1, 2, 3 or 4;
• -S-(CH₂)ₙ-, with n being 1, 2, 3 or 4;
• (CH₂)ₙ-(CHR⁵)-(CH₂)ₘ, with n and m being selected from 0, 1, 2 or 3 and m+n being 1, 2 or 3, with R⁵ being selected from F, Cl, Br, I, OH, SH, or unsubstituted C₁₋₄-Alkyl;
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants.

18. Medicament according to claim 17, comprising at least one compound according to formula la, 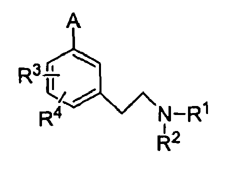 , wherein
A is a compound selected from the following group 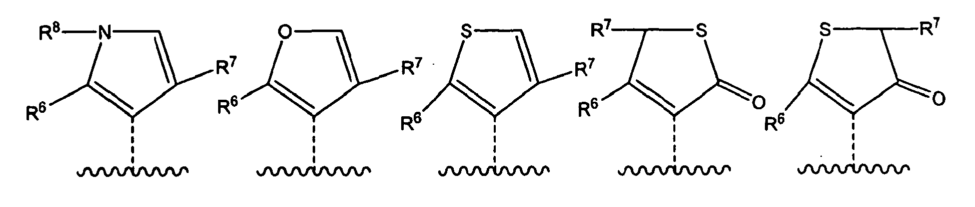 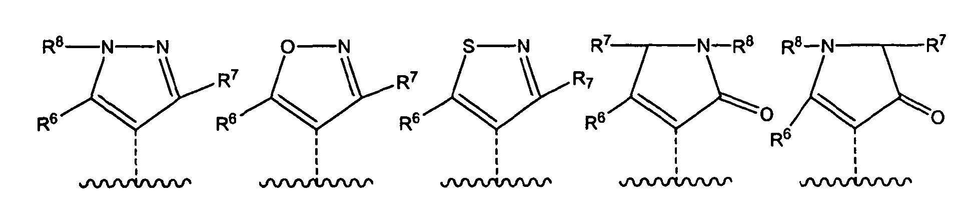 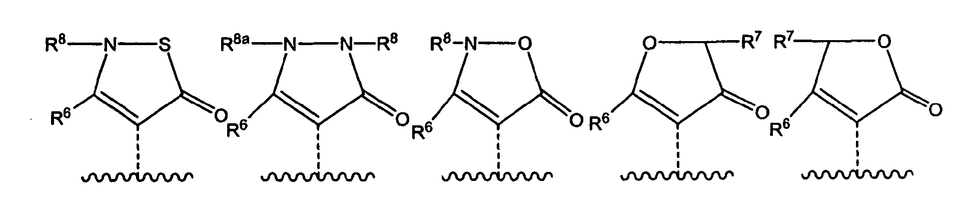 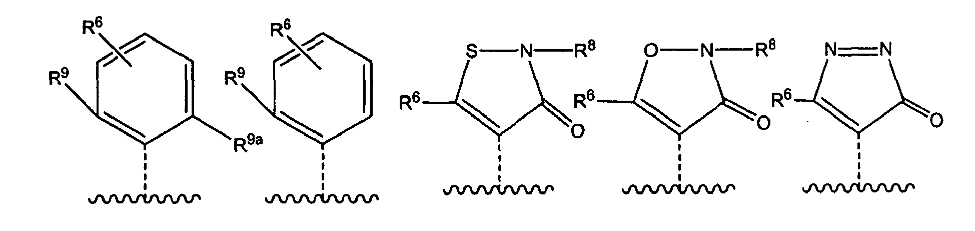
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants.

19. Medicament according to any of claims 17 or 18, comprising at least one compound according to formula la, 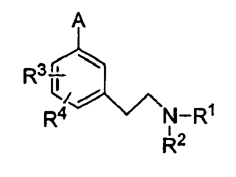 , wherein
A is a compound selected from the following group 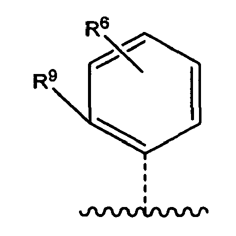 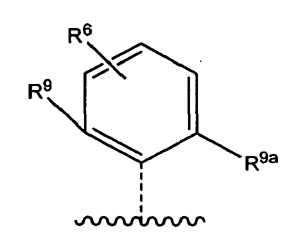
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ is selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants.

20. Medicament comprising at least one compound according to claims 1-14, optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, and optionally one or more pharmaceutically acceptable adjuvants.

21. Use of at least one compound according to formula I, 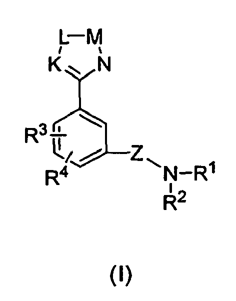 wherein
K-L-M-N together form
• =CH-X-Y=CH-; in which any suitable H may be substituted by R⁶ and/or R⁷, and in which X is selected from NR⁸, O or S, while Y is selected from N or CH;
• =CH-X-Y-C(O)-; in which any suitable H may be substituted by R⁶ and in which one of X and Y is NR⁸, while the other is selected from NR^{8a}, S or O;
• =CH-X-Y-C(O)-; in which one of X and Y is CH₂, while the other is selected from NR⁸, S or O, in which any suitable H may be substituted by R⁶ and/or R⁷;
• =CR⁶-N=N-C(O)-;
• =CR⁹-CH=CH-CH=CH-; in which any suitable H may be substituted by R⁶;
• =CR⁹-CH=CH-CH=CR^{9a}-; in which any suitable H may be substituted by R⁶;
• =CH-X=Y-CH=CH-; in which any suitable H may be substituted by R⁶ and/or R⁷, and in which one of X or Y is selected from N, while the other is selected from N or CH;
• =CH-X=Y-CH₂-CH₂-; in which any suitable H may be substituted by R⁶ and/or R⁷ and in which one of X or Y is selected from N, while the other is selected from N or CH;
• =CH-X-Y-CH=CH-; in which any suitable H may be substituted by R⁶ and/or R⁷, and in which one of X or Y is selected from NR⁸, O or S while the other is selected from NR^{8a} or CH₂;
• =CH-X-Y-CH₂-CH₂-; in which any suitable H may be substituted by R⁶ and/or R⁷, and in which one of X or Y is selected from NR⁸, O or S while the other is selected from NR^{8a} or CH₂;
• =CH-X-CH₂-Y=CH-; in which any suitable H may be substituted by R⁶ and/or R⁷, and in which X is selected from NR⁸, O or S while Y is selected from N or CH;
• =CH-X-CH=Y-CH₂-; in which any suitable H may be substituted by R⁶ and/or R⁷, and in which X is selected from NR⁸, O or S while Y is selected from N or CH;
• =CH-N=CH-Y=CH-; in which any suitable H may be substituted by R⁶ and/or R⁷;
• =CH-X-CH₂-Y-CH₂-; in which any suitable H may be substituted by R⁶ and/or R⁷, and in which one of X or Y is selected from NR⁸, O or S while the other is selected from NR^{8a}, O, S or CH₂;
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem;
Z is selected from
• -(CH₂)ₙ-, with n being 1, 2, 3 or 4;
• -O-(CH₂)ₙ-, with n being 1, 2, 3 or 4;
• -S-(CH₂)ₙ-, with n being 1, 2, 3 or 4;
• (CH₂)ₙ-(CHR⁵)-(CH₂)ₘ, with n and m being selected from 0, 1, 2 or 3 and m+n being 1, 2 or 3, with R⁵ being selected from F, Cl, Br, I, OH, SH, or unsubstituted C₁₋₄-Alkyl;
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the treatment of a 5-HT₇ mediated disease or condition.

22. Use according to claim 21 of at least one compound according to formula la, 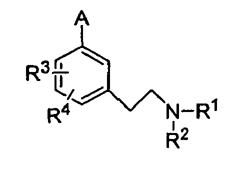 , wherein
A is a compound selected from the following group 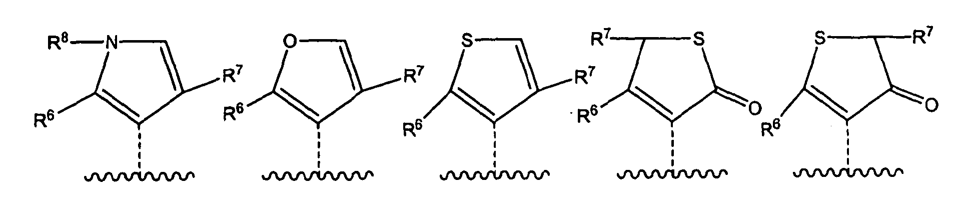 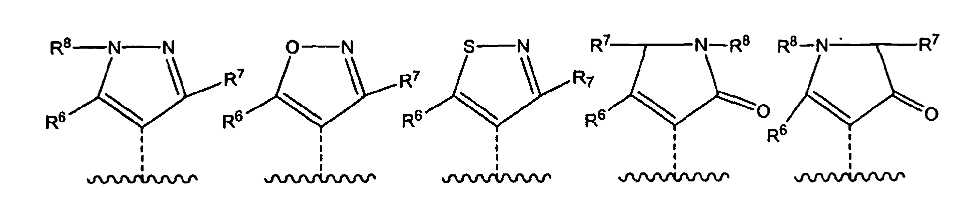 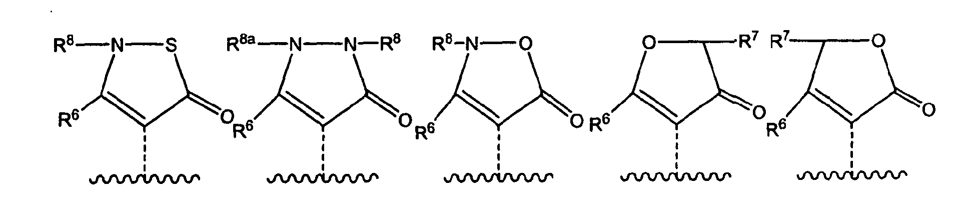 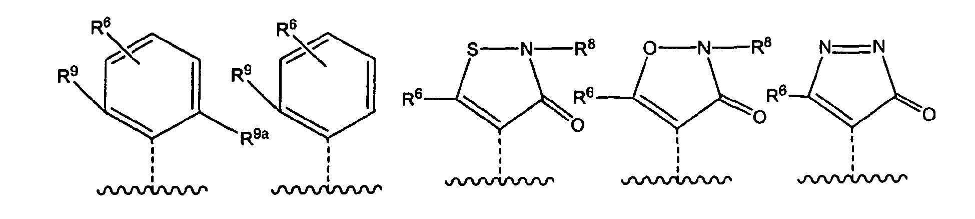
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ and R⁷ are independently from each other selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁸ and R^{8a} are independently from each other selected from hydrogen; or an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the treatment of a 5-HT₇ mediated disease or condition.

23. Use according to any of claims 21 or 22 of at least one compound according to formula la, 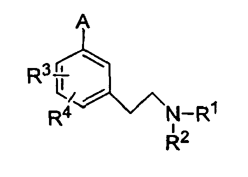 , wherein
A is a compound selected from the following group 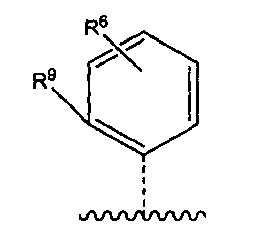 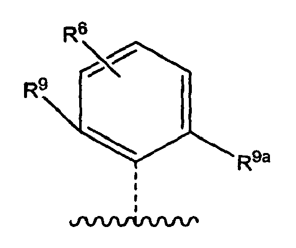
R¹ and R² each are independently selected from the group consisting of hydrogen; or a linear or branched, saturated or unsaturated, optionally at least mono-substituted aliphatic radical; or
R¹ and R² together with the bridging nitrogen atom form an saturated or unsaturated, optionally at least mono-substituted 5- or 6-membered-heterocyclic ring, which may be condensed with an optionally at least mono-substituted mono- or polycyclic ringsystem,
R³ and R⁴ are independently from each other selected from hydrogen; halogen, OH, SH, NH₂; a linear or branched, saturated or unsaturated, optionally at least mono-substituted aliphatic radical; or O-R with R being a linear or branched, saturated or unsaturated, optionally at least mono-substituted aliphatic radical;
R⁶ is selected from hydrogen; halogen, OH, SH, NH₂; an aliphatic radical, which is linear or branched, saturated or unsaturated, and optionally at least mono-substituted by F, Cl, Br, I, SH or OH; or O-R with R being an aliphatic radical, which is linear or branched, saturated or unsaturated, and optionally at least mono-substituted by F, Cl, Br, I, SH or OH;
R⁹ and R^{9a} are independently from each other selected from an aliphatic radical, which is linear or branched, saturated or unsaturated, and optionally at least mono-substituted by F, Cl, Br, I, SH or OH; ; or O-R with R being an aliphatic radical, which is linear or branched, saturated or unsaturated, and optionally at least mono-substituted by F, Cl, Br, I, SH or OH;
optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the treatment of a 5-HT₇ mediated disease or condition.

24. Use of at least one compound according to claims 1-14, optionally in form of one of its stereoisomers, preferably enantiomers or diastereomers, its racemate or in form of a mixture of at least two of its stereoisomers in any mixing ratio, or a physiologically acceptable salt thereof, or a solvate, respectively, for the manufacture of a medicament for the treatment of a 5-HT₇ mediated disease or condition.

25. Use according to any of claims 21 to 24 wherein the disease is pain, preferably visceral pain, chronic pain, cancer pain, migraine, acute pain or neuropathic pain, more prefearably neuropathic pain, allodynia or hyperalgesia.

26. Use according to any of claims 21 to 24 wherein the disease is sleep disorder, shift worker syndrome, jet lag, depression, seasonal affective disorder, migraine, anxiety, psychosis, schizophrenia, cognition and memory disorders, neuronal degeneration resulting from ischemic events, cardiovascular diseases such as hypertension, irritable bowel syndrome, inflammatory bowel disease, spastic colon or urinary incontinence.
